(19) Europäisches Patentamt
European Patent Office
Office européen des brevets

(11) **EP 2 155 382 B1**

(12) **EUROPÄISCHE PATENTSCHRIFT**

(45) Veröffentlichungstag und Bekanntmachung des Hinweises auf die Patenterteilung:
**24.04.2019 Patentblatt 2019/17**

(21) Anmeldenummer: **08758899.2**

(22) Anmeldetag: **30.05.2008**

(51) Int Cl.:
**B01J 23/38** *(2006.01)*    **B01J 35/00** *(2006.01)*
**B01J 37/00** *(2006.01)*

(86) Internationale Anmeldenummer:
**PCT/EP2008/004328**

(87) Internationale Veröffentlichungsnummer:
**WO 2008/145388 (04.12.2008 Gazette 2008/49)**

(54) **VERFAHREN ZUR HERSTELLUNG EINES SCHALENKATALYSATORS, SCHALENKATALYSATOR UND DESSEN VERWENDUNG**

METHOD FOR PRODUCING A SHELL CATALYST, CORRESPONDING SHELL CATALYST AND ITS USE

PROCÉDÉ DE PRODUCTION D'UN CATALYSEUR SOUS ENVELOPPE, CATALYSEUR SOUS ENVELOPPE ET SON UTILISATION

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **31.05.2007 DE 102007025442**

(43) Veröffentlichungstag der Anmeldung:
**24.02.2010 Patentblatt 2010/08**

(73) Patentinhaber: **Süd-Chemie IP GmbH & Co. KG 81925 München (DE)**

(72) Erfinder:
• **HAGEMEYER, Alfred**
  **83043 Bad Aibling (DE)**
• **MESTL, Gerhard**
  **80935 München (DE)**
• **SCHECK, Peter**
  **82205 Gilching (DE)**
• **UNGAR, Sybille**
  **81541 München (DE)**

(74) Vertreter: **Kuba, Stefan et al Clariant Produkte (Deutschland) GmbH IPM / Patent & License Management Arabellastraße 4a 81925 München (DE)**

(56) Entgegenhaltungen:
WO-A-99/62632    WO-A-02/100527
WO-A-2005/061107    WO-A-2006/027009

EP 2 155 382 B1

**Beschreibung**

Die vorliegende Erfindung betrifft ein Verfahren zur Herstellung eines Schalenkatalysators, der einen porösen Katalysatorträger-Formkörper mit einer äußeren Schale umfasst, in welcher zumindest eine katalytisch aktive Spezies enthalten ist.

Schalenkatalysatoren und Verfahren zu deren Herstellung sind im Stand der Technik bekannt. In Schalenkatalysatoren sind die katalytisch wirkenden Spezies - häufig auch die Promotoren - nur in einem mehr oder weniger breiten äußeren Bereich (Schale) eines Katalysatorträger-Formkörpers enthalten, d.h. sie durchdringen den Katalysatorträger-Formkörper nicht vollständig (vgl. beispielsweise EP 565 952 A1, EP 634 214 A1, EP 634 209 A1 und EP 634 208 A1). Mit Schalenkatalysatoren ist in vielen Fällen eine selektivere Reaktionsführung möglich als mit Katalysatoren, bei denen der Träger bis in den Trägerkern hinein mit der katalytisch aktiven Spezies beladen ("durchimprägniert") ist.

Vinylacetat-Monomer (VAM) beispielsweise wird gegenwärtig überwiegend mittels Schalenkatalysatoren in hoher Selektivität hergestellt. Der überwiegende Anteil der derzeit eingesetzten Schalenkatalysatoren zur Herstellung von VAM sind Schalenkatalysatoren mit einer Pd/Au-Schale auf einem porösen amorphen, als Kugel ausgebildeten Alumosilikatträger auf der Basis von natürlichen Schichtsilikaten, die mit Kaliumacetat als Promotor durchimprägniert sind. In dem Pd/Au-System dieser Katalysatoren liegen die Aktivmetalle Pd und Au vermutlich nicht in Form von Metallpartikeln des jeweiligen reinen Metalls vor, sondern vielmehr in Form von Pd/Au-Legierungspartikeln von möglicherweise unterschiedlicher Zusammensetzung, wenngleich das Vorliegen von unlegierten Partikeln nicht ausgeschlossen werden kann.

VAM-Schalenkatalysatoren werden üblicherweise auf so genanntem chemischen Wege hergestellt, bei welchem der Katalysatorträger mit Lösungen von entsprechenden Metallverbindungen beispielsweise durch Eintauchen des Trägers in die Lösungen oder mittels des Incipient-Wetness-Verfahrens (Porenfüllverfahren), bei welchem der Träger mit einem seinem Porenvolumen entsprechenden Lösungsvolumen beladen wird, getränkt wird.

Die WO 99/62632 A1 beschreibt einen Katalysator für die Herstellung von Vinylacetat mittels Reaktion von Ethylen, Sauerstoff und Essigsäure, umfassend einen porösen Träger, auf dessen porösen Oberflächen katalytisch effektive Mengen an metallischem Palladium und Gold abgeschieden sind. Dieser Katalysator wurde durch Imprägnieren eines porösen Trägers, dessen porösen Oberflächen eine katalytisch effektive Menge an vorreduziertem metallischem Palladium enthalten, mit einer Lösung von Kaliumaurat, gefolgt von der Reduzierung des Kaliumaurats in eine katalytisch effektive Menge metallischen Goldes.

In der WO 2005/061107 A1 werden Palladium- und Gold-Precursoren auf einen zirkoniumhaltigen Träger aufgebracht. Dies erfolgt insbesondere durch Imprägnierung der entsprechenden Lösungen mittels Incipient-Wetness-Verfahren.

Die Pd/Au-Schale eines VAM-Schalenkatalysators wird beispielsweise erzeugt, indem zunächst der Katalysatorträger-Formkörper in einem ersten Schritt mit einer $Na_2PdCl_4$-Lösung getränkt und anschließend in einem zweiten Schritt die Pd-Komponente mit NaOH-Lösung auf den Katalysatorträger in Form einer Pd-Hydroxidverbindung fixiert wird. In einem darauffolgenden separaten dritten Schritt wird der Katalysatorträger dann mit einer $NaAuCl_4$-Lösung getränkt und danach die Au-Komponente ebenfalls mittels NaOH fixiert. Es ist beispielsweise auch möglich, den Träger zunächst mit Lauge zu tränken und dann die Vorläuferverbindungen auf den so vorbehandelten Träger aufzubringen. Nach der Fixierung der Edelmetallkomponenten am Katalysatorträger wird der beladene Katalysatorträger dann weitestgehend frei von Chlorid- und Na-Ionen gewaschen, anschließend getrocknet und abschließend bei 150 °C mit Ethylen reduziert. Die erzeugte Pd/Au-Schale weist üblicherweise eine Dicke von etwa 100 bis 500 $\mu$m auf, wobei in der Regel die Produktselektivität eines Schalenkatalysators umso höher ist, je geringer die Dicke seiner Schale ist.

Üblicherweise nach dem Fixierungs- oder Reduzierungsschritt wird der mit den Edelmetallen beladene Katalysatorträger dann mit Kaliumacetat beladen, wobei die Beladung mit Kaliumacetat nicht nur in der äußeren, mit Edelmetallen beladenen Schale erfolgt, sondern der Katalysatorträger vielmehr mit dem Promotor vollständig durchimprägniert wird.

Nach dem Stand der Technik werden die Aktivmetalle Pd und Au ausgehend von Chloridverbindungen im Bereich einer Schale des Trägers auf demselben mittels Tränken aufgebracht. Diese Technik ist jedoch an die Grenzen angelangt, was minimale Schalendicken und maximale Au-Beladung angeht. Die dünnste erreichbare Schalendicke entsprechend hergestellter VAM-Katalysatoren liegt bei bestenfalls ca. 100 $\mu$m und es ist nicht absehbar, dass mittels Tränkung noch dünnere Schalen erhalten werden können. Darüber hinaus weisen die mittels Tränkung hergestellten Katalysatoren uneinheitliche Schalendicken auf sowie über verhältnismäßig große Bereiche ihrer Schalendicke hinweg eine sehr uneinheitliche Konzentration an katalytisch aktiver Spezies, was sich nachteilig auf die Produktselektivität sowie auf die Aktivität des Katalysators auswirken kann.

Aufgabe der vorliegenden Erfindung ist es daher, ein Schalenkatalysator-Herstellungsverfahren bereitzustellen, mittels welchem Schalenkatalysatoren herstellbar sind, die über verhältnismäßig große Bereiche ihrer Schalendicke hinweg eine weitgehend einheitliche Konzentration an katalytisch aktiver Spezies sowie eine weitgehend einheitliche Schalendicke aufweisen.

Diese Aufgabe wird durch ein Verfahren unter Nutzung einer Vorrichtung gelöst, die eingerichtet ist, mittels eines Prozessgases ein Fließbett von Katalysatorträger-Formkörpern zu erzeugen, in welchem die Katalysatorträger-Formkörper elliptisch oder toroidal umlaufen, vorzugsweise toroidal, wobei das Verfahren die nachfolgenden Schritte umfasst

a) Beschicken der Vorrichtung (10) mit porösen Katalysatorträger-Formkörpern und Erzeugen eines Katalysatorträger-Formkörper-Fließbettes mittels eines Prozessgases (40), wobei die Katalysatorträger-Formkörper in dem Fließbett elliptisch oder toroidal umlaufen, vorzugsweise toroidal;

b) Besprühen der Katalysatorträger-Formkörper mit Lösung enthaltend eine katalytisch aktive Spezies oder einen Vorläufer davon, wobei die Katalysatorträger-Formkörper in dem Fließbett elliptisch oder toroidal umlaufen;

c) Trocknen der mit der Lösung besprühten Katalysatorträger-Formkörper.

Überraschenderweise wurde festgestellt, dass mittels des erfindungsgemäßen Verfahrens Schalenkatalysatoren herstellbar sind, die über große Bereiche ihrer Schalendicke hinweg eine weitgehend einheitliche Konzentration an katalytisch aktiver Spezies sowie eine weitgehend einheitliche Schalendicke aufweisen. Darüber hinaus lassen sich mittels des erfindungsgemäßen Verfahrens Katalysatoren mit sehr dünnen Schalen herstellen, beispielsweise kleiner 100 $\mu$m.

Darüber hinaus zeichnen sich die mittels des erfindungsgemäßen Verfahrens hergestellten Schalenkatalysatoren im Vergleich zu mittels im Stand der Technik bekannter Verfahren hergestellten Katalysatoren durch eine erhöhte Aktivität aus.

Soll der Schalenkatalysator in der Schale mehrere voneinander verschiedene katalytisch aktive Spezies enthalten, beispielsweise mehrere Aktivmetalle oder ein Aktivmetall und ein Promotormetall, so kann der Katalysatorträger-Formkörper dem erfindungsgemäßen Verfahren entsprechend häufig unterworfen werden. Alternativ dazu kann das erfindungsgemäße Verfahren auch mit Mischlösungen durchgeführt werden, welche die gewünschten, voneinander verschiedenen katalytisch aktiven Spezies oder Vorläufer davon enthalten. Ferner können gemäß des erfindungsgemäßen Verfahrens die Katalysatorträger gleichzeitig mit voneinander verschiedenen Lösungen verschiedener katalytisch aktiver Spezies oder Vorläufer davon besprüht werden.

Mittels des erfindungsgemäßen Verfahrens sind Schalenkatalysatoren herstellbar, deren Konzentration an katalytisch aktiver Spezies über einen Bereich von 90 % der Schalendicke hinweg, wobei der Bereich zur äußeren und inneren Schalengrenze jeweils um 5 % der Schalendicke beabstandet ist, von der mittleren Konzentration dieses Bereichs an katalytisch aktiver Spezies von +/- 3 bis maximal +/- 20 % abweicht.

Darüber hinaus hat das erfindungsgemäße Verfahren den Vorteil, dass sich mittels diesem Verfahren Chargen mit einer Vielzahl von Schalenkatalysatoren herstellen lassen, deren Verhältnis von der Standardabweichung ihrer Schalendicke zum Mittelwert der Schalendicke kleiner/gleich 20 % ist. Derartige Werte werden mittels der im Stand der Technik bekannten Verfahren zur Herstellung von Schalenkatalysatoren nicht erreicht.

Die Trocknung der mit der Lösung besprühten Formkörper erfolgt im Rahmen des erfindungsgemäßen Verfahrens vorzugsweise kontinuierlich mittels des Prozessgases. Es kann aber auch vorgesehen sein, dass nach Imprägnierung unter kontinuierlicher Trocknung ein gesonderter abschließender Trocknungsschritt durchgeführt wird. Im ersten Fall, beispielsweise, kann durch die Temperatur des Prozessgases bzw. der Formkörper die Trocknungsgeschwindigkeit und damit die Eindringtiefe (Dicke der Schale) individuell eingestellt werden, im zweiten Fall kann durch jede dem Fachmann als geeignet bekannte Trocknungsmethode getrocknet werden.

Vorrichtungen zur Durchführung des erfindungsgemäßen Verfahrens sind beispielsweise in den Dokumenten WO 2006/027009 A1, DE 102 48 116 B3, EP 0 370 167 A1, EP 0 436 787 B1, DE 199 04 147 A1, DE 20 2005 003 791 U1 beschrieben, deren Inhalt mittels Referenzierung in die vorliegende Erfindung miteinbezogen wird.

So beschreibt zum Beispiel die WO 2006/027009 A1 eine Vorrichtung zum Behandeln von partikelförmigem Gut, mit einer Prozesskammer zum Aufnehmen und zum Behandeln des Gutes, die einen Boden aufweist, der aus mehreren übereinandergelegten, sich einander überlappenden, ringförmigen Leitplatten aufgebaut ist, zwischen denen ringförmige Schlitze ausgebildet sind, über die Prozessluft mit einer im Wesentlichen horizontalen, radial nach außen gerichteten Bewegungskomponente einführbar ist.

Gegenstand der WO 02/100527 A1 ist eine Vorrichtung zum Behandeln von partikelförmigem Gut, mit einer Prozesskammer zum Aufnehmen und zum Behandeln des Gutes, wobei ein Boden der Prozesskammer aus einander überlappenden Leitplatten aufgebaut ist, zwischen denen Schlitze gebildet sind, über die Prozessluft mit einer im wesentlichen horizontalen Bewegungskomponente in die Prozesskammer einführbar ist, wobei die Schlitze derart angeordnet sind, dass zwei aufeinander zu gerichtete Strömungen entstehen, die längs einer Aufbruchzone aufeinandertreffen und in eine vertikal nach oben gerichtete Strömung umgelenkt werden.

Weitere geeignete und erfindungsgemäß bevorzugte Fließbettanlagen werden von den Unternehmen Glatt GmbH (Binzen, Deutschland), Aeromatic-Fielder AG (Bubendorf, Schweiz), Fluid Air Inc. (Aurora, Illinois, USA), Hüttlin GmbH (Steinen, Deutschland), Umang Pharmatech Pvt. Ltd. (Marharashtra, Indien) und Innojet Technologies (Lörrach, Deutschland) vertrieben. Zur Durchführung des erfindungsgemäßen Verfahrens besonders bevorzugte Fließbettvorrichtungen werden von der Firma Innojet Technologies unter den Bezeichnungen Innojet® Ventilus oder Innojet® Air-Coater vertrieben. Diese Vorrichtungen umfassen einen zylindrischen Behälter mit einem fest und unbeweglich eingebauten Behälterboden, in dessen Mitte eine Sprühdüse montiert ist. Der Boden besteht aus kreisrunden Lamellen, die stufenweise übereinander montiert sind. Die Prozessluft strömt zwischen den einzelnen Lamellen waagerecht exzentrisch mit einer umfänglichen Strömungskomponente nach außen in Richtung der Behälterwand in den Behälter ein. Dabei bilden sich so genannte Luftgleitschichten aus, auf denen die Katalysatorträger-Formkörper, zunächst nach außen in Richtung Behälterwand transportiert werden. Außen an der Behälterwand ist ein senkrecht ausgerichteter Prozessluftstrom installiert, der die Katalysatorträger nach oben umlenkt. Oben angekommen bewegen sich die Katalysatorträger auf einer tangentialen Bahn in Richtung Mitte des Bodens zurück, in dessen Verlauf sie den Sprühnebel der Düse passieren. Nach dem passieren des Sprühnebels beginnt der beschriebene Bewegungsvorgang von Neuem. Die beschriebene Prozessluftführung liefert dabei die Grundlage für eine weitgehend homogene toroidale fließbettartige Umwälzbewegung der Katalysatorträger.

Das Zusammenwirken des Besprühens mit der fließbettartigen elliptischen oder toroidalen Umwälzbewegung der Katalysatorträger im Fließbett bewirkt im Gegensatz zu einer entsprechenden herkömmlichen Wirbelschicht, dass die einzelnen Katalysatorträger in annähernd gleicher Häufigkeit die Sprühdüse passieren. Darüber hinaus sorgt der Umwälzvorgang auch dafür, dass die einzelnen Katalysatorträger eine Rotation um ihre eigene Achse durchführen, weshalb die Katalysatorträger besonders gleichmäßig imprägniert werden.

In dem erfindungsgemäßen Verfahren wird ein Fliessbett erzeugt, in welchem die Formkörper elliptisch oder toroidal umlaufen. Im Stand der Technik wird der Übergang der Partikel einer Schüttung in einen Zustand, in welchen die Partikel vollständig frei beweglich werden (Fließbett), als Lockerungspunkt (Wirbelpunkt) bezeichnet und die entsprechende Fluidgeschwindigkeit als Lockerungsgeschwindigkeit. Erfindungsgemäß bevorzugt ist es, dass in dem erfindungsgemäßen Verfahren die Fluidgeschwindigkeit bis zum 4-fachen der Lockerungsgeschwindigkeit beträgt, bevorzugt bis zum drei-fachen der Lockerungsgeschwindigkeit und mehr bevorzugt bis zum 2-fachen der Lockerungsgeschwindigkeit.

Gemäß einer alternativen Ausführungsform des erfindungsgemäßen Verfahrens kann es vorgesehen sein, dass die Fluidgeschwindigkeit bis zum 1,4-fachen des Zehnerlogarithmus der Lockerungsgeschwindigkeit beträgt, bevorzugt bis zum 1,3-fachen des Zehnerlogarithmus der Lockerungsgeschwindigkeit und mehr bevorzugt bis zum 1,2-fachen des Zehnerlogarithmus der Lockerungsgeschwindigkeit.

Die Begriffe "Katalysatorträger-Formkörper", "Katalysatorträger", "Formkörper" und "Träger" werden im Rahmen der vorliegenden Erfindung synonym verwendet.

In dem erfindungsgemäßen Verfahren laufen die Katalysatorträger-Formkörper in dem Fließbett elliptisch oder toroidal um, vorzugsweise toroidal. Um eine Vorstellung davon zu geben, wie sich die Formkörper in dem Fließbett bewegen, sei ausgeführt, dass bei "elliptischem Umlaufen" sich die Katalysatorträger-Formkörper in dem Fließbett in vertikaler Ebene auf einer elliptischen Bahn bewegen mit wechselnder Größe der Haupt- und Nebenachse. Bei "toroidalem Umlaufen" bewegen sich die Katalysatorträger-Formkörper in dem Fließbett in vertikaler Ebene auf einer elliptischen Bahn mit wechselnder Größe der Haupt- und Nebenachse und in horizontaler Ebene auf einer Kreisbahn mit wechselnder Größe des Radius. Im Mittel bewegen sich die Formkörper bei "elliptischem Umlaufen" in vertikaler Ebene auf einer elliptischen Bahn, bei "toroidalem Umlaufen" auf einer toroidalen Bahn, d.h., dass ein Formkörper die Oberfläche eines Torus mit vertikal elliptischem Schnitt helikal abfährt.

Für den Fall, dass das erfindungsgemäße Verfahren mittels einer Lösung eines Vorläufers einer katalytisch aktiven Spezies durchgeführt wird, umfasst das Verfahren ferner einen Schritt des Überführens des Vorläufers in die entsprechende katalytisch aktive Spezies. Das Überführen des Vorläufers kann dabei mit Hilfe jeder Methode durchgeführt werden, die dem Fachmann als für das erfindungsgemäße Verfahren geeignet bekannt ist.

Zur Bewerkstelligung eines Katalysatorträger-Formkörper-Fließbettes, in welchem die Katalysatorträger-Formkörper elliptisch oder toroidal umlaufen, auf verfahrenstechnisch einfache und damit kostengünstige Weise ist entsprechend einer weiter bevorzugten Ausführungsform des erfindungsgemäßen Verfahrens vorgesehen, dass die Vorrichtung eine Prozesskammer mit einem Boden und eine Seitenwand umfasst, wobei das Prozessgas durch den Boden der Prozesskammer, der vorzugsweise aus mehreren übereinandergelegten, sich einander überlappenden ringförmigen Leitplatten aufgebaut ist, zwischen denen ringförmige Schlitze ausgebildet sind, mit einer horizontalen, radial nach außen gerichteten Bewegungskomponente in die Prozesskammer eingeführt wird.

Dadurch, dass Prozessgas mit einer horizontalen, radial nach außen gerichteten Bewegungskomponente in die Prozesskammer eingeführt wird, wird ein elliptisches Umlaufen der Katalysatorträger in dem Fließbett bewirkt. Sollen die Formkörper in dem Fließbett toroidal umlaufen, so muss den Formkörpern zusätzlich noch eine umfängliche Bewegungskomponente auferlegt werden, welche die Formkörper auf eine Kreisbahn zwingt. Diese umfängliche Bewegungskomponente kann den Formkörpern beispielsweise auferlegt werden, indem an der Seitenwand entsprechend ausgerichtete Führungsschienen zur Umlenkung der Katalysatorträger angeordnet sind. Gemäß einer weiter bevorzugten Ausführungsform des erfindungsgemäßen Verfahrens ist es jedoch vorgesehen, dass dem in die Prozesskammer eingeführten Prozessgas eine umfängliche Strömungskomponente auferlegt wird. Dadurch wird die Erzeugung des Katalysatorträger-Formkörper-Fließbettes, in welchem die Katalysatorträger-Formkörper toroidal umlaufen, verfahrenstechnisch einfach gewährleistet.

Um dem in die Prozesskammer eingeführten Prozessgas die umfängliche Strömungskomponente aufzuerlegen, kann es gemäß einer bevorzugten Ausführungsform des erfindungsgemäßen Verfahrens vorgesehen sein, dass zwischen den ringförmigen Leitplatten entsprechend geformte und ausgerichtete Prozessgas-Leitelemente angeordnet sind. Alternativ oder zusätzlich dazu kann es vorgesehen sein, dass dem in die Prozesskammer eingeführten Prozessgas die umfängliche Strömungskomponente auferlegt wird, indem durch den Boden der Prozesskammer zusätzliches Prozessgas mit einer schräg nach oben gerichteten Bewegungskomponente in die Prozesskammer eingeführt wird, vorzugsweise im Bereich der Seitenwand der Prozesskammer.

Es kann vorgesehen sein, dass das Besprühen der in dem Fließbett umlaufenden Katalysatorträger-Formkörper mit der Lösung mittels einer Ringspaltdüse durchgeführt wird, die eine Sprühwolke versprüht, wobei die Symmetrieebene der Sprühwolke vorzugsweise parallel zur Ebene des Gerätebodens verläuft. Durch den 360°- Umfang der Sprühwolke können die sich mittig herabbewegenden Formkörper besonders gleichmäßig mit der Lösung besprüht werden. Dabei ist die Ringspaltdüse, d.h. deren Mündung, vorzugsweise vollständig in dem Fließbett eingebettet.

Entsprechend einer weiter bevorzugten Ausführungsform des erfindungsgemäßen Verfahrens ist es vorgesehen, dass die Ringspaltdüse mittig im Boden angeordnet ist und die Mündung der Ringspaltdüse in dem Fließbett vollständig eingebettet ist. Dadurch wird gewährleistet, dass die freie Weglänge der Tropfen der Sprühwolke bis zum Auftreffen auf einen Formkörper verhältnismäßig kurz ist und entsprechend den Tropfen verhältnismäßig wenig Zeit verbleibt, zu größeren Tropfen zu koaleszieren, was der Ausbildung einer weitgehend einheitlichen Schalendicke entgegenwirken könnte.

Gemäß einer weiter bevorzugten Ausführungsform des erfindungsgemäßen Verfahrens kann es vorgesehen sein, dass an der Unterseite der Sprühwolke ein Gasstützpolster bewerkstelligt wird. Das bodenseitige Polster hält die Bodenoberfläche weitgehend frei von versprühter Lösung, das heißt, dass nahezu die gesamte versprühte Lösung in das Fließbett der Formkörper eingetragen wird, so dass kaum Sprühverluste auftreten, was insbesondere hinsichtlich teurer Edelmetalle/-verbindungen oder Enzyme aus Kostengründen von Bedeutung ist.

Entsprechend einer weiter bevorzugten Ausführungsform des erfindungsgemäßen Verfahrens ist es vorgesehen, dass der Katalysatorträger kugelförmig ausgebildet ist. Dadurch wird eine gleichmäßige Rotation des Trägers um seine Achse und damit einhergehend eine gleichmäßige Imprägnierung des Katalysatorträgers mit der Lösung der katalytisch aktiven Spezies gewährleistet.

Als Katalysatorträger können in dem erfindungsgemäßen Verfahren sämtliche poröse Katalysatorträger-Formkörper eingesetzt werden, die sich mittels eines Prozessgases elliptisch oder toroidal umwälzen lassen, wobei die Träger aus sämtlichen Materialien oder Materialmischungen gebildet sein können. Erfindungsgemäß bevorzugt sind jedoch solche Katalysatorträger, die zumindest ein Metalloxid umfassen oder aus einem solchen oder einer Mischung davon gebildet sind. Vorzugsweise umfasst der Katalysatorträger ein Siliziumoxid, ein Aluminiumoxid, ein Alumosilikat, ein Zirkoniumoxid, ein Titanoxid, ein Nioboxid oder ein natürliches Schichtsilikat, vorzugsweise einen kalzinierten säurebehandelten Bentonit.

Unter dem Begriff "natürliches Schichtsilikat", wofür in der Literatur auch der Begriff "Phyllosilikat" verwendet wird, wird aus natürlichen Quellen stammendes unbehandeltes oder behandeltes Silikat-Mineral verstanden, in welchem $SiO_4$- Tetraeder, welche die strukturelle Grundeinheit aller Silikate bilden, in Schichten der allgemeinen Formel $[Si_2O_5]^{2-}$ miteinander vernetzt sind. Diese Tetraederschichten wechsellagern mit so genannten Oktaederschichten, in denen ein Kation, vor allem Al und Mg, oktaedrisch von OH bzw. O umgeben ist. Dabei werden beispielsweise Zweischicht-Phyllosilikate und Dreischicht-Phyllosilikate unterschieden. Im Rahmen der vorliegenden Erfindung bevorzugte Schichtsilikate sind Tonminerale, insbesondere Kaolinit, Beidellit, Hectorit, Saponit, Nontronit, Glimmer, Vermiculit und Smektite, wobei Smektite und dabei insbesondere Montmorillonit besonders bevorzugt sind. Definitionen des Begriffes "Schichtsilikate" finden sich beispielsweise in "Lehrbuch der anorganischen Chemie", Hollemann Wiberg, de Gruyter, 102. Auflage, 2007 (ISBN 978-3-11-017770-1) oder in "Römpp Lexikon Chemie", 10. Auflage, Georg Thieme Verlag unter dem Begriff "Phyllosilikat". Typische Behandlungen, denen ein natürliches Schichtsilikat vor dem Einsatz als Trägermaterial unterzogen wird, beinhalten beispielsweise ein Behandeln mit Säuren und/oder ein Kalzinieren. Ein im Rahmen der vorliegenden Erfindung besonders bevorzugtes natürliches Schichtsilikat ist ein Bentonit. Bentonite sind zwar im eigentlichen Sinne keine natürlichen Schichtsilikate, sondern vielmehr ein Gemisch von überwiegend Tonmineralien, in welchem Schichtsilikate enthalten sind. Vorliegend ist also für den Fall, dass das natürliche Schichtsilikat ein Bentonit ist, zu verstehen, dass das natürliche Schichtsilikat in dem Katalysatorträger in Form oder als Bestandteil eines Bentonits vorliegt.

Ein als Formkörper ausgebildeter Katalysatorträger auf der Basis von natürlichen Schichtsilikaten, insbesondere auf der Basis eines säurebehandelten kalzinierten Bentonits, kann beispielsweise hergestellt werden, indem eine einen säurebehandelten (unkalzinierten) Bentonit als Schichtsilikat und Wasser enthaltende Formenmischung unter Verdichtung zu einem Formkörper mittels dem Fachmann geläufiger Vorrichtungen, wie beispielsweise Extrudern oder Tablettenpressen, geformt wird und anschließend der nicht ausgehärtete Formkörper zu einem stabilen Formkörper kalziniert wird. Dabei hängt die Größe der spezifischen Oberfläche des Katalysatorträgers insbesondere von der Qualität des eingesetzten (Roh-)Bentonits ab, dem Säurebehandlungsverfahren des eingesetzten Bentonits, d.h. beispielsweise der Natur und der zum Bentonit relativen Menge und der Konzentration der eingesetzten anorganischen Säure, der Säurebehandlungsdauer sowie der -temperatur, vom Verpressungsdruck sowie von der Kalzinierdauer und - temperatur sowie der Kalzinieratmosphäre.

Säurebehandelte Bentonite können durch Behandlung von Bentoniten mit starken Säuren erhalten werden, wie beispielsweise Schwefelsäure, Phosphorsäure oder Salzsäure. Eine auch im Rahmen der vorliegenden Erfindung geltende Definition des Begriffes Bentonit ist in Römpp, Lexikon Chemie, 10. Aufl., Georg Thieme Verlag, angegeben. Im Rahmen der vorliegenden Erfindung besonders bevorzugte Bentonite sind natürliche aluminiumhaltige Schichtsilikate, die Montmorillonit (als Smektit) als Hauptmineral enthalten. Nach der Säurebehandlung wird der Bentonit in der Regel mit Wasser gewaschen, getrocknet und zu einem Pulver vermahlen.

Es konnte festgestellt werden, dass mittels des erfindungsgemäßen Verfahrens auch verhältnismäßig große Schalendicken erreicht werden können. Und zwar ist die erzielbare Dicke der Schale umso größer, je kleiner die Oberfläche des Trägers ist. Gemäß einer weiter bevorzugten Ausführungsform des erfindungsgemäßen Verfahrens kann vorgesehen sein, dass der Katalysatorträger eine Oberfläche von kleiner/gleich 160 m$^2$/g aufweist, vorzugsweise eine von kleiner als 140 m$^2$/g, bevorzugt eine von kleiner als 135 m$^2$/g, weiter bevorzugt eine von kleiner als 120 m$^2$/g, mehr bevorzugt eine von kleiner als 100 m$^2$/g, noch mehr bevorzugt eine von kleiner als 80 m$^2$/g und besonders bevorzugt eine von kleiner als 65 m$^2$/g. Unter dem Begriff "Oberfläche" des Katalysatorträgers wird dabei im Rahmen der vorliegenden Erfindung die BET-Oberfläche des Trägers verstanden, die mittels Adsorption von Stickstoff nach DIN 66132 bestimmt wird.

Entsprechend einer weiter bevorzugten Ausführungsform des erfindungsgemäßen Verfahrens ist es vorgesehen, dass der Katalysatorträger eine Oberfläche von 160 bis 40 m$^2$/g aufweist, vorzugsweise eine von zwischen 140 und 50 m$^2$/g, bevorzugt eine von zwischen 135 und 50 m$^2$/g, weiter bevorzugt eine von zwischen 120 und 50 m$^2$/g, mehr bevorzugt eine von zwischen 100 und 50 m$^2$/g und am meisten bevorzugt eine von zwischen 100 und 60 m$^2$/g.

Beim Umwälzen der Träger im Rahmen des erfindungsgemäßen Verfahrens werden die Katalysatorträger mechanisch beansprucht, wodurch es zu einem gewissen Abrieb sowie einer gewissen Beschädigung von Katalysatorträgern, insbesondere im Bereich der entstehenden Schale, kommen kann. Insbesondere um den Abrieb des Katalysatorträgers in vertretbaren Grenzen zu halten, weist der Katalysatorträger eine Härte von größer/gleich 20 N auf, vorzugsweise eine von größer/gleich 30 N, weiter bevorzugt eine von größer/gleich 40 N und am meisten bevorzugt eine von größer/gleich 50 N. Die Ermittlung der Härte ist dabei mittels eines Tablettenhärtetesters 8M der Fa. Dr. Schleuniger Pharmatron AG an 99 Stück Formkörpern als Durchschnitt bestimmt nach Trocknung bei 130 °C für 2 h, wobei die Geräteeinstellungen wie folgt sind:

| | |
|---|---|
| Härte: | N |
| Distanz zum Formkörper: | 5,00 mm |
| Zeitverzögerung: | 0,80 s |
| Vorschub-Typ: | 6 D |
| Geschwindigkeit: | 0,60 mm/s |

Die Härte des Katalysatorträgers kann beispielsweise mittels Variation gewisser Parameter des Verfahrens zu seiner Herstellung beeinflusst werden, beispielsweise durch die Auswahl des Trägermaterials, die Kalzinierdauer und/oder die Kalziniertemperatur eines aus einer entsprechenden Trägermischung geformten, unausgehärteten Formkörpers, oder durch bestimmte Zuschlagsstoffe wie beispielsweise Methylcellulose oder Magnesiumstearat.

Aus Kostengründen wird in dem erfindungsgemäßen Verfahren als Prozessgas vorzugsweise Luft eingesetzt. Sollte jedoch beispielsweise die katalytisch aktive Spezies oder der Vorläufer davon mit Luftsauerstoff zu unerwünschten Verbindungen reagieren, kann es auch vorgesehen sein, dass als Prozessgas ein Inertgas eingesetzt wird, beispielsweise Stickstoff, Methan, kurzkettige gesättigte Kohlenwasserstoffe, eines der Edelgase, vorzugsweise Helium, Neon oder Argon, oder ein halogenierter Kohlenwasserstoff oder eine Mischung von zwei oder mehr der vorgenannten.

Gemäß einer weiteren bevorzugten Ausführungsform des erfindungsgemäßen Verfahrens kann das Prozessgas, vor allem im Falle teurer Gase wie z.B. Helium, Argon etc., in einem geschlossenen Kreislauf in die Vorrichtung rückgeführt werden.

Gemäß einer weiter bevorzugten Ausführungsform des erfindungsgemäßen Verfahrens wird der Katalysatorträger vor und/oder während des Auftragens der Lösung erwärmt, beispielsweise mittels eines erwärmten Prozessgases. Über den Grad der Erwärmung der Katalysatorträger kann die Abtrocknungsgeschwindigkeit der aufgetragenen Lösung der katalytisch aktiven Spezies bestimmt werden. Bei relativ niedrigen Temperaturen beispielsweise ist die Abtrocknungsgeschwindigkeit verhältnismäßig klein, so dass es bei entsprechendem quantitativem Auftrag aufgrund der durch das Vorhandensein von Lösungsmittel bedingten hohen Diffusion der aktiven Spezies oder des Vorläufers davon zur Ausbildung größerer Schalendicken kommen kann. Bei relativ hohen Temperaturen beispielsweise ist die Abtrocknungsgeschwindigkeit verhältnismäßig hoch, so dass mit dem Katalysatorträger in Kontakt kommende Lösung nahezu unverzüglich abtrocknet, weshalb auf dem Katalysatorträger aufgetragene Lösung nicht tief in denselben eindringen kann.

Bei verhältnismäßig hohen Temperaturen können so Schalen mit relativ kleinen Dicken und hoher Beladung an aktiver Spezies erhalten werden. Entsprechend wird gemäß einer weiter bevorzugten Ausführungsform des erfindungsgemäßen Verfahrens das Prozessgas erwärmt, vorzugsweise auf eine Temperatur von größer/gleich 40 °C, bevorzugt auf eine Temperatur von größer/gleich 60 °C, weiter bevorzugt auf eine Temperatur von größer/gleich 70 °C und am meisten bevorzugt auf eine Temperatur von 60 bis 100 °C.

Um ein vorzeitiges Abtrocknen von Tropfen der Sprühwolke zu verhindern, kann es gemäß einer weiter bevorzugten Ausführungsform des erfindungsgemäßen Verfahrens vorgesehen sein, dass das Prozessgas vor der Einführung in die Vorrichtung mit dem Lösungsmittel der Lösung angereichert wird, vorzugsweise in einem Bereich von 10 bis 50 % des Sättigungsdampfdrucks (bei Prozesstemperatur).

Gemäß einer weiteren bevorzugten Ausführungsform des erfindungsgemäßen Verfahrens kann das dem Prozessgas zugegebene Lösungsmittel sowie Lösungsmittel von der Abtrocknung der Formkörper mittels geeigneter Kühleraggregate, Kondensatoren und Abscheider vom Prozessgas abgetrennt und mittels einer Pumpe in den Lösungsmittelanreicherer rückgeführt werden.

Es kann bevorzugt sein, dass die Lösung der katalytisch aktiven Spezies die Lösung eines Biokatalysators ist, vorzugsweise die Lösung eines Enzyms. Insbesondere Enzymlösungen lassen sich mittels des erfindungsgemäßen Verfahrens schnell und einfach zu Schalenkatalysatoren verarbeiten.

In das erfindungsgemäße Verfahren können auch Lösungen von Metallverbindungen beliebiger Metalle eingesetzt werden. Bevorzugt ist es jedoch, wenn die Lösung eine Lösung einer Metallverbindung eines Metalls ist, ausgewählt aus der Gruppe bestehend aus den Übergangsmetallen, insbesondere den Edelmetallen.

Ferner kann es entsprechend einer weiter bevorzugten Ausführungsform des erfindungsgemäßen Verfahrens vorgesehen sein, dass die Metallverbindungen ausgewählt sind aus den Halogeniden, insbesondere Chloriden, Oxiden, Nitraten, Nitriten, Formiaten, Propionaten, Oxalaten, Acetaten, Hydroxiden, Hydrogencarbonaten, Aminkomplexen oder organischen Komplexen, beispielsweise Triphenylphosphinkomplexen oder Acetylacetonatkomplexen, der genannten Metalle.

Zur Herstellung eines Schalenkatalysators für Oxidationsreaktionen ist es entsprechend einer weiter bevorzugten Ausführungsform des erfindungsgemäßen Verfahrens vorgesehen, wenn die Lösung eine Lösung einer Pd-Vorläuferverbindung ist.

Zur Herstellung eines silberhaltigen Schalenkatalysators ist es entsprechend einer weiter bevorzugten Ausführungsform des erfindungsgemäßen Verfahrens vorgesehen, wenn die Lösung der katalytisch aktiven Spezies oder eines Vorläufers davon eine Lösung einer Ag-Verbindung ist.

Zur Herstellung eines platinhaltigen Schalenkatalysators ist es entsprechend einer weiter bevorzugten Ausführungsform des erfindungsgemäßen Verfahrens vorgesehen, wenn die Lösung eine Lösung einer Pt-Vorläuferverbindung ist.

Zur Herstellung eines goldhaltigen Schalenkatalysators ist es entsprechend einer weiter bevorzugten Ausführungsform des erfindungsgemäßen Verfahrens vorgesehen, wenn die Lösung der katalytisch aktiven Spezies oder eines Vorläufers davon eine Lösung einer Au-Verbindung ist.

Entsprechend kann es zur Herstellung eines nickel-, kobalt- oder kupferhaltigen Schalenkatalysators entsprechend einer weiter bevorzugten Ausführungsform des erfindungsgemäßen Verfahrens vorgesehen sein, wenn die Lösung der katalytisch aktiven Spezies oder eines Vorläufers davon eine Lösung einer Nickel-, Kobalt- bzw. Kupferverbindung ist.

In den im Stand der Technik beschriebenen Verfahren zur Herstellung von VAM-Schalenkatalysatoren auf der Basis von Pd und Au werden üblicherweise kommerziell erhältliche Lösungen der Vorläuferverbindungen wie $Na_2PdCl_4$-, $NaAuCl_4$- oder $HAuCl_4$-Lösungen eingesetzt. In der jüngeren Literatur werden auch chloridfreie Pd- oder Au-Vorläuferverbindungen wie beispielsweise $Pd(NH_3)_4(OH)_2$, $Pd(NH_3)_2(NO_2)_2$ und $KAuO_2$ eingesetzt. Diese Vorläuferverbindungen reagieren in Lösung basisch, während die klassischen Chlorid-, Nitrat- und Acetat-Vorläuferverbindungen in Lösung allesamt sauer reagieren.

Grundsätzlich kann als Pd- und Au-Vorläuferverbindung jede Pd- bzw. Au-Verbindung eingesetzt werden, mittels der ein hoher Dispersionsgrad der Metalle erzielt werden kann. Dabei wird unter dem Begriff "Dispersionsgrad" das Verhältnis der Anzahl aller Oberflächenmetallatome aller Metall-/Legierungspartikel eines geträgerten Metallkatalysators zu der Gesamtzahl aller Metallatome der Metall-/Legierungspartikel verstanden. Im Allgemeinen ist es bevorzugt, wenn der Dispersionsgrad einem verhältnismäßig hohen Zahlenwert entspricht, da in diesem Fall möglichst viele Metallatome für eine katalytische Reaktion frei zugänglich sind. Das heißt, dass bei einem verhältnismäßig hohen Dispersionsgrad eines geträgerten Metallkatalysators eine bestimmte katalytische Aktivität desselben mit einer verhältnismäßig geringen Menge an eingesetztem Metall erreicht werden kann.

Beispiele für bevorzugte Pd-Vorläuferverbindungen sind wasserlösliche Pd-Salze. Gemäß einer besonders bevorzugten Ausführungsform des erfindungsgemäßen Verfahrens ist die Pd-Vorläuferverbindung ausgewählt aus der Gruppe bestehend aus $Pd(NH_3)_4(OH)_2$, $Pd(NH_3)_4(OAc)_2$, $Pd(NH_3)_4(HCO_3)_2$, $Pd(NH_3)_4(HPO_4)$, $Pd(NH_3)_4Cl_2$, $Pd(NO_3)_2$, $K_2Pd(OAc)_2(OH)_2$, $Na_2Pd(OAc)_2(OH)_2$, $Pd(NH_3)_2(NO_2)_2$, $Pd(NH_3)_4(NO_3)_2$, $K_2Pd(NO_2)_4$, $Na_2Pd(NO_2)_4$, $Pd(OAc)_2$, $PdCl_2$, $H_2PdCl_4$, $(NH_4)_2PdCl_4$, $K_2PdCl_4$ und $Na_2PdCl_4$. Neben $Pd(OAc)_2$ können auch andere Carboxylate des Palladiums eingesetzt werden, vorzugsweise die Salze der aliphatischen Monocarbonsäuren mit 3 bis 5 Kohlenstoffatomen, beispielsweise das Propionat- oder das Butyratsalz. Anstelle von $NH_3$ können auch die entsprechenden Pd-Verbindungen mit Ethylendiamin oder Ethanolamin als Ligand eingesetzt werden.

Entsprechend einer weiter bevorzugten Ausführungsform des erfindungsgemäßen Verfahrens können auch Pd-Nitrit-Vorläuferverbindungen bevorzugt sein. Bevorzugte Pd-Nitrit-Vorläuferverbindungen sind beispielsweise solche, die mittels Lösen von $Pd(OAc)_2$ in einer $NaNO_2$-Lösung erhalten werden.

Beispiele für bevorzugte Au-Vorläuferverbindungen sind wasserlösliche Au-Salze. Gemäß einer besonders bevorzugten Ausführungsform des erfindungsgemäßen Verfahrens ist die Au-Vorläuferverbindung ausgewählt aus der Gruppe bestehend aus $KAuO_2$, $NaAuO_2$, $NMe_4AuO_2$, $KAuCl_4$, $(NH_4)AuCl_4$, $HAuCl_4$, $KAu(NO_2)_4$, $NaAu(NO_2)_4$, $AuCl_3$, $NaAuCl_4$, $KAu(OAc)_3(OH)$, $NaAu(OAc)_3(OH)$, $HAu(NO_3)_4$ und $Au(OAc)_3$. Dabei ist es gegebenenfalls empfehlenswert, das $Au(OAc)_3$ oder das $KAuO_2$ mittels Fällung des Oxids/Hydroxids aus einer Goldsäure-Lösung, Waschung und Isolierung des Niederschlags sowie Aufnahme desselben in Essigsäure bzw. KOH jeweils frisch anzusetzen.

Beispiele für bevorzugte Pt-Vorläuferverbindungen sind wasserlösliche Pt-Salze. Gemäß einer besonders bevorzugten Ausführungsform des erfindungsgemäßen Verfahrens ist die Pt-Vorläuferverbindung ausgewählt aus der Gruppe bestehend aus $Pt(NH_3)_4(OH)_2$, $K_2PtCl_4$, $K_2PtCl_6$, $Na_2PtCl_6$, $Pt(NH_3)_4Cl_2$, $Pt(NH_3)_4(HCO_3)_2$, $Pt(NH_3)_4(HPO_4)$, $Pt(NO_3)_2$, $K_2Pt(OAc)_2(OH)_2$, $Pt(NH_3)_2(NO_2)_2$, $PtCl_4$, $H_2Pt(OH)_6$, $Na_2Pt(OH)_6$, $K_2Pt(OH)_6$, $K_2Pt(NO_2)_4$, $Na_2Pt(NO_2)_4$, $Pt(OAc)_2$, $PtCl_2$ und $Na_2PtCl_4$. Neben $Pt(OAc)_2$ können auch andere Carboxylate des Platins eingesetzt werden, vorzugsweise die Salze der aliphatischen Monocarbonsäuren mit 3 bis 5 Kohlenstoffatomen, beispielsweise das Propionat- oder das Butyratsalz.

Entsprechend einer weiter bevorzugten Ausführungsform des erfindungsgemäßen Verfahrens können auch Pt-Nitrit-Vorläuferverbindungen bevorzugt sein. Bevorzugte Pt-Nitrit-Vorläuferverbindungen sind beispielsweise solche, die mittels Lösen von $Pt(OAc)_2$ in einer $NaNO_2$-Lösung erhalten werden.

Beispiele für bevorzugte Ag-Vorläuferverbindungen sind wasserlösliche Ag-Salze. Gemäß einer besonders bevorzugten Ausführungsform des erfindungsgemäßen Verfahrens ist die Ag-Vorläuferverbindung ausgewählt aus der Gruppe bestehend aus $Ag(NH_3)_2(OH)$, $Ag(NO_3)$, Ag-Citrat, Ag-Tartrat, Ammonium-Ag-Oxalat, $K_2Ag(OAc)(OH)_2$, $Ag(NH_3)_2(NO_2)$, $Ag(NO_2)$, Ag-lactat, Agtrifluoracetat, Ag-Oxalat, $Ag_2CO_3$, $K_2Ag(NO_2)_3$, $Na_2Ag(NO_2)_3$, $Ag(OAc)$, ammoniakalische AgCl-Lösung oder ammoniakalische $Ag_2CO_3$-Lösung oder ammoniakalische AgO-Lösung. Neben $Ag(OAc)$ können auch andere Carboxylate des Silbers eingesetzt werden, vorzugsweise die Salze der aliphatischen Monocarbonsäuren mit 3 bis 5 Kohlenstoffatomen, beispielsweise das Propionat- oder das Butyratsalz. Anstelle von $NH_3$ können auch die entsprechenden Ethylendiamine oder andere Diamine von Ag eingesetzt werden.

Entsprechend einer weiter bevorzugten Ausführungsform des erfindungsgemäßen Verfahrens können auch Ag-Nitrit-Vorläuferverbindungen bevorzugt sein. Bevorzugte Ag-Nitrit-Vorläuferverbindungen sind beispielsweise solche, die mittels Lösen von $Ag(OAc)$ in einer $NaNO_2$-Lösung erhalten werden.

Als Lösungsmittel für metallische, katalytisch aktive Spezies oder deren Vorläufer sind alle Lösungsmittel geeignet, in denen die ausgewählte Metallverbindung löslich ist und die nach dem Auftrag auf den Katalysatorträger von demselben mittels Trocknung leicht wieder entfernt werden können. Bevorzugte Lösungsmittel-Beispiele für Metallacetate als Vorläuferverbindungen sind vor allem unsubstituierte Carbonsäuren, insbesondere Essigsäure, oder Ketone wie Aceton und für die Metallchloride vor allem Wasser oder verdünnte Salzsäure.

Falls die Vorläuferverbindung in Essigsäure, Wasser bzw. verdünnter Salzsäure oder Mischungen davon nicht ausreichend löslich ist, können alternativ oder zusätzlich zu den genannten Lösungsmitteln auch andere Lösungsmittel Anwendung finden. Als andere Lösungsmittel kommen hierbei vorzugsweise diejenigen Lösungsmittel in Betracht, die inert sind. Als bevorzugte Lösungsmittel, die sich als Zusatz zur Essigsäure eignen, seien Ketone, beispielsweise Aceton oder Acetylaceton, ferner Ether, beispielsweise Tetrahydrofuran oder Dioxan, Acetonitril, Dimethylformamid und Lösungsmittel auf der Basis von Kohlenwasserstoffen wie beispielsweise Benzol genannt.

Als bevorzugte Lösungsmittel oder Additive, die sich als Zusatz zu Wasser eignen, seien Ketone, beispielsweise Aceton, oder Alkohole, beispielsweise Ethanol oder Isopropanol oder Methoxyethanol, Laugen, wie wässrige KOH oder NaOH, oder organische Säuren, wie Essigsäure, Ameisensäure, Zitronensäure, Weinsäure, Äpfelsäure, Glyoxylsäure, Glycolsäure, Oxalsäure, Benztraubensäure oder Milchsäure, genannt.

Bevorzugt ist es, wenn im Rahmen des erfindungsgemäßen Verfahrens das in den Prozess eingesetzte Lösungsmittel rückgewonnen wird, vorzugsweise mittels geeigneter Kühleraggregate, Kondensatoren und Abscheider.

Entsprechend einer weiter bevorzugten Ausführungsform des erfindungsgemäßen Verfahrens ist vorgesehen, dass der Katalysatorträger-Formkörper nach dem Besprühen mit der Lösung der katalytisch aktiven Spezies oder eines Vorläufers davon einem Fixierungsschritt unterworfen wird zur Fixierung der katalytisch aktiven Spezies oder des Vorläufers auf dem Katalysatorträger. Der Fixierungsschritt kann dabei beispielsweise im Hinblick auf Edelmetalle die Behandlung des Trägers mit Lauge beinhalten, z.B. durch Aufsprühen von Base auf den Träger in der Vorrichtung (Fließbettvorrichtung) oder durch eine Kalzinierung des Trägers zur Überführung der Metallkomponenten der entsprechenden Metallverbindungen in eine Hydroxid-Verbindung bzw. in ein Oxid.

Im Falle von Übergangsmetallverbindungen, die beispielsweise mittels einer Base auf dem Katalysatorträger fixiert worden sind, kann der Träger nachträglich zur Überführung der MetallKomponente der Metallverbindung in die entsprechende Oxidform kalziniert werden. Nach dem Beladen mit den Vorläuferverbindungen bzw. nach der Fixierung der Metall-Komponenten kann der Träger - zur Überführung der Metall-Komponenten in die entsprechenden Oxide - kalziniert werden. Die Kalzinierung erfolgt bevorzugt bei Temperaturen von weniger als 1000 °C.

Zur Herstellung geträgerter Übergangsmetall- bzw. Edelmetallkatalysatoren in Form von Schalenkatalysatoren werden die Metall-Komponenten vor dem Einsatz des Katalysators noch reduziert, wobei die Reduktion in situ, d.h. im Prozessreaktor, oder auch ex situ, d.h. in einem speziellen Reduktionsreaktor, durchgeführt werden kann. Die Reduktion in situ wird vorzugsweise mit Ethylen (5 Vol.-%) in Stickstoff bei einer Temperatur von etwa 150 °C über einen Zeitraum von beispielsweise 5 Stunden durchgeführt. Die Reduktion ex situ kann beispielsweise mit 5 Vol.-% Wasserstoff in Stickstoff, beispielsweise mittels Formiergas, bei Temperaturen im Bereich von vorzugsweise 150-500 °C über einen Zeitraum von 5 Stunden durchgeführt werden.

Gasförmige oder verdampfbare Reduktionsmittel wie beispielsweise CO, $NH_3$, Formaldehyd, Methanol und Kohlenwasserstoffe können ebenfalls eingesetzt werden, wobei die gasförmigen Reduktionsmittel auch mit Inertgas, wie beispielsweise Kohlendioxid, Stickstoff oder Argon, verdünnt sein können. Vorzugsweise wird ein mit Inertgas verdünntes Reduktionsmittel eingesetzt. Bevorzugt sind Mischungen von Wasserstoff mit Stickstoff oder Argon, vorzugsweise mit einem Wasserstoffgehalt zwischen 1 Vol.-% und 15 Vol.-%.

Die Reduktion der Übergangsmetalle oder Edelmetalle kann auch in flüssiger Phase vorgenommen werden, vorzugsweise mittels der Reduktionsmittel Hydrazin, K-Formiat, Na-Formiat, Ameisensäure, $H_2O_2$, hypophophorige Säure oder Na-hypophosphit.

Die Menge an Reduktionsmittel wird vorzugsweise so gewählt, dass während der Behandlungsdauer zumindest das zur vollständigen Reduktion der Metall-Komponenten nötige Äquivalent über den Katalysator geleitet wird. Bevorzugt wird jedoch ein Überschuss an Reduktionsmittel über den Katalysator geleitet, um eine schnelle und vollständige Reduktion zu gewährleisten.

Vorzugsweise wird drucklos, d.h. bei einem Absolutdruck von ca. 1 bar, reduziert. Für die Herstellung technischer Mengen an erfindungsgemäßem Katalysator wird bevorzugt ein Drehrohrofen, Fließbett- oder Wirbelschichtreaktor verwendet, um eine gleichmäßige Reduktion des Katalysators zu gewährleisten.

Die vorliegende Erfindung betrifft ferner einen Schalenkatalysator, umfassend einen porösen Katalysatorträger-Formkörper mit einer äußeren Schale, in welcher zumindest eine katalytisch aktive Spezies enthalten ist, wobei die Konzentration der katalytisch aktiven Spezies über einen Bereich von 90 % der Schalendicke hinweg, wobei der Bereich zur äußeren und inneren Schalengrenze jeweils um 5 % der Schalendicke beabstandet ist, von der mittleren Konzentration an katalytisch aktiver Spezies dieses Bereichs um maximal +/-20 % abweicht, vorzugsweise um maximal +/- 15 % und bevorzugt um maximal +/- 10 %. Derartige Schalenkatalysatoren sind mittels des erfindungsgemäßen Verfahrens erhältlich.

Ist die katalytisch aktive Spezies und/oder der Promotor ein Metall, so lässt sich dessen/deren Verteilung im Träger ermitteln, indem ein Schnitt des Katalysators hergestellt wird, beispielsweise indem der Träger halbiert wird. Im Elektronenmikroskop lässt sich dann die räumliche Verteilung des Aktivmetalls bzw. des Promotormetalls mit Hilfe der WDX-Spektroskopie (Wellenlängendispersive Röntgenbeugung) ermitteln, die auch als EDX-Spektroskopie (Energy Dispersive X-ray) bezeichnet wird. Dabei wird ein Messkopf über die Probe hinweg geführt, der sensitiv auf das Aktivmetall, bevorzugt Palladium, bzw. das Promotormetall, bevorzugt Gold, ist, sodass deren Verteilung in der Fläche ermittelt werden kann.

Durch die weitgehend einheitliche Verteilung der katalytisch aktiven Spezies innerhalb der Schale wird eine weitgehend einheitliche Aktivität des erfindungsgemäßen Katalysators über die Dicke der Schale hinweg gewährleistet, da die Konzentration an aktiver Spezies über die Schalendicke hinweg nur verhältnismäßig wenig variiert. D.h., dass das Profil der Konzentration an aktiver Spezies über die Schalendicke hinweg annähernd eine Rechteckfunktion beschreibt.

Zur weiteren Erhöhung der Selektivität weist der erfindungsgemäße Katalysator das Merkmal auf, dass über die Dicke der Schale des Katalysators hinweg gesehen die maximale Konzentration an katalytisch aktiver Spezies im Bereich der äußeren Schalengrenze liegt und die Konzentration in Richtung der inneren Schalengrenze abfällt. Dabei kann es bevorzugt sein, wenn die Konzentration an katalytisch aktiver Spezies in Richtung der inneren Schalengrenze über einen Bereich von zumindest 25 % der Schalendicke hinweg stetig abfällt, vorzugsweise über einen Bereich von zumindest 40 % der Schalendicke und bevorzugt über einen Bereich von 30 bis 80 % der Schalendicke.

Entsprechend einer weiter bevorzugten Ausführungsform des erfindungsgemäßen Katalysators fällt die Konzentration an katalytisch aktiver Spezies in Richtung der inneren Schalengrenze auf eine Konzentration von 50 bis 90 % der maximalen Konzentration in etwa stetig ab, vorzugsweise auf eine Konzentration von 70 bis 90 % der maximalen Konzentration.

Ein nicht erfindungsgemäßer Gegenstand betrifft ferner einen Schalenkatalysator, umfassend einen porösen Katalysatorträger-Formkörper sowie zumindest eine katalytisch aktive Spezies, die in einer äußeren Schale des Katalysatorträger-Formkörpers enthalten ist, wobei der Schalenkatalysator ein Element einer Charge einer Vielzahl von Schalenkatalysatoren ist, wobei das Verhältnis der Standardabweichung der Schalendicken der Schalenkatalysatoren der Charge zum Mittelwert der Schalendicken der Schalenkatalysatoren der Charge kleiner/gleich 20 % ist, vorzugsweise kleiner/gleich 15 %, bevorzugt kleiner/gleich 12 % und mehr bevorzugt kleiner/gleich 10 % oder 3 bis 18 %, vorzugsweise 3 bis 15 %.

Die Standardabweichung ist dabei entsprechend der Formel

$$\sigma_X = \sqrt{\frac{1}{N-1}\sum_{i=1}^{N}(X_i - \overline{X})^2}$$

ermittelt, in welcher

$\sigma_x$ die Standardabweichung ist;

N (= 100) der Stichprobenumfang ist (Anzahl der Katalysatorträger-Formkörper; N ist gleich 100);

$X_i$ die Schalendicke am i-ten Katalysatorträger-Formkörper der Stichprobe ist;

$\overline{X}$ der empirische Mittelwert der Schalendicke der Stichprobe (also das arithmetische Mittel der Stichprobe) ist, der sich entsprechend der Formel

$$\overline{X} = \frac{1}{N}\sum_{i=1}^{N}X_i$$

bestimmt. Derartige Schalenkatalysator-Chargen sind mittels des erfindungsgemäßen Verfahrens herstellbar.

Es kann vorgesehen sein, dass die katalytisch aktive Spezies ein Biokatalysator ist, vorzugsweise ein Enzym. Der erfindungsgemäße Schalenkatalysator kann alternativ dazu ein Metall oder jegliche Kombination von Metallen enthalten, die sich als katalytisch aktive Metalle oder als Promotormetall eignen. Dabei kann das Metall beispielsweise in metallischer Form, in ionischer Form oder beispielsweise in komplexierter Form vorliegen. Bevorzugt ist es, wenn das Metall ausgewählt ist aus der Gruppe der Übergangsmetalle, vorzugsweise aus der Gruppe der Edelmetalle.

Erfindungsgemäß bevorzugte Katalysatoren enthalten zwei voneinander verschiedene Metalle in metallischer Form in der Schale, wobei die beiden Metalle Kombinationen einer der folgenden Paare sind: Pd und Ag; Pd und Au; Pd und Pt. Katalysatoren mit einer Pd/Au-Schale eignen sich insbesondere zur Herstellung von VAM, die mit einer Pd/Pt-Schale eignen sich insbesondere als Oxidations- und Hydrierkatalysator und die mit einer Pd/Ag-Schale eignen sich insbesondere zur selektiven Hydrierung von Alkinen und Dienen in Olefinströmen, also beispielweise zur Herstellung von gereinigtem Ethylen durch selektive Hydrierung von im Rohprodukt enthaltenem Acetylen.

Hinsichtlich der Bereitstellung eines VAM-Schalenkatalysators mit ausreichender VAM-Aktivität ist es bevorzugt, dass der Katalysator als katalytisch aktive Spezies Pd und Au enthält und der Anteil des Katalysators an Pd 0,6 bis 2,0 Mass.-% beträgt, vorzugsweise 0,7 bis 1,8 Mass.-% und bevorzugt 0,8 bis 1,5 Mass.-% bezogen auf die Masse des mit Edelmetall beladenen Katalysatorträgers.

Darüber hinaus ist es in vorgenanntem Zusammenhang bevorzugt, dass das Au/Pd-Atomverhältnis des Katalysators zwischen 0 und 1,2 beträgt, vorzugsweise zwischen 0,1 und 1, bevorzugt zwischen 0,3 und 0,9 und besonders bevorzugt zwischen 0,4 und 0,8.

Im Falle eines Pd/Au-Schalenkatalysators enthält dieser vorzugsweise als Promotor zumindest eine Alkalimetallverbindung, vorzugsweise eine Kalium-, eine Natrium-, eine Cäsium- oder eine Rubidiumverbindung, bevorzugt eine Kaliumverbindung. Zu den geeigneten und besonders bevorzugten Kaliumverbindungen gehören Kaliumacetat KOAc, Kaliumcarbonat $K_2CO_3$, Kaliumhydrogencarbonat $KHCO_3$ und Kaliumhydroxid KOH sowie sämtliche Kaliumverbindungen, die sich unter den jeweiligen Reaktionsbedingungen der VAM-Synthese in K-Acetat KOAc umwandeln. Die Kaliumverbindung kann sowohl vor als auch nach der Reduktion der Metall-Komponenten zu den Metallen Pd und Au auf den Katalysatorträger aufgetragen werden. Entsprechend einer weiter bevorzugten Ausführungsform des erfindungsgemäßen Katalysators umfasst der Katalysator ein Alkalimetallacetat, vorzugsweise Kaliumacetat. Dabei ist es zur Gewährleistung einer ausreichenden Promotoraktivität besonders bevorzugt, wenn der Gehalt des Katalysators an Alkalimetallacetat 0,1 bis 0,7 mol/l beträgt, vorzugsweise 0,3 bis 0,5 mol/l.

Entsprechend einer weiter bevorzugten Ausführungsform des erfindungsgemäßen Pd/Au-Katalysators beträgt das Alkalimetall/Pd-Atomverhältnis zwischen 1 und 12, vorzugsweise zwischen 2 und 10 und besonders bevorzugt zwischen 4 und 9. Dabei ist vorzugsweise das Alkalimetall/Pd-Atomverhältnis umso geringer, je kleiner die Oberfläche des Katalysatorträgers ist.

Es wurde festgestellt, dass die Produktselektivitäten des erfindungsgemäßen Pd/Au-Katalysators umso höher sind, je kleiner die Oberfläche des Katalysatorträgers ist. Darüber hinaus kann die Dicke der Metallschale umso größer gewählt sein, je kleiner die Oberfläche des Katalysatorträgers ist, ohne nennenswerte Verluste an Produktselektivität hinnehmen zu müssen. Gemäß einer bevorzugten Ausführungsform des erfindungsgemäßen Katalysators weist die Oberfläche des Katalysatorträgers daher eine Oberfläche von kleiner/gleich 160 m$^2$/g auf, vorzugsweise eine von kleiner als 140 m$^2$/g, bevorzugt eine von kleiner als 135 m$^2$/g, weiter bevorzugt eine von kleiner als 120 m$^2$/g, mehr bevorzugt eine von kleiner als 100 m$^2$/g, noch mehr bevorzugt eine von kleiner als 80 m$^2$/g und besonders bevorzugt eine von kleiner als 65 m$^2$/g.

Entsprechend einer weiter bevorzugten Ausführungsform des erfindungsgemäßen Pd/Au-Katalysators kann es vorgesehen sein, dass der Katalysatorträger eine Oberfläche von 160 bis 40 m$^2$/g aufweist, vorzugsweise eine von zwischen 140 und 50 m$^2$/g, bevorzugt eine von zwischen 135 und 50 m$^2$/g, weiter bevorzugt eine von zwischen 120 und 50 m$^2$/g, mehr bevorzugt eine von zwischen 100 und 50 m$^2$/g und am meisten bevorzugt eine von zwischen 100 und 60 m$^2$/g.

Im Hinblick auf eine geringe Porendiffusionslimitierung kann gemäß einer weiteren bevorzugten Ausführungsform des erfindungsgemäßen Pd/Au-Katalysators vorgesehen sein, dass der Katalysatorträger einen mittleren Porendurchmesser von 8 bis 50 nm aufweist, vorzugsweise einen von 10 bis 35 nm und bevorzugt einen von 11 bis 30 nm.

Die Azidität des Katalysatorträgers kann die Aktivität des erfindungsgemäßen Katalysators vorteilhaft beeinflussen. Entsprechend einer weiter bevorzugten Ausführungsform des erfindungsgemäßen Katalysators besitzt der Katalysatorträger eine Azidität von zwischen 1 und 150 μval/g, vorzugsweise eine von zwischen 5 und 130 μval/g und besonders bevorzugt eine von zwischen 10 und 100 μval/g. Die Azidität des Katalysatorträgers wird dabei wie folgt bestimmt: 1 g des fein gemahlenen Katalysatorträgers wird mit 100 ml Wasser (mit einem pH-Blindwert) versetzt und unter Rühren 15 Minuten extrahiert. Anschließend wird mit 0,01 n NaOH-Lösung zumindest bis pH 7,0 titriert, wobei die Titration stufenweise erfolgt; und zwar wird zunächst 1 ml der NaOH-Lösung zu dem Extrakt getropft (1 Tropfen/Sekunde), dann 2 Minuten gewartet, der pH-Wert abgelesen, erneut 1 ml NaOH zugetropft, usw. Der Blindwert des eingesetzten Wassers wird bestimmt und die Azidität-Berechnung entsprechend korrigiert.

Die Titrationskurve (ml 0,01 NaOH gegen pH-Wert) wird dann aufgetragen und der Schnittpunkt der Titrationskurve bei pH 7 bestimmt. Berechnet werden die Moläquivalente in 10$^{-6}$ äquiv/g Träger, die sich aus dem NaOH-Verbrauch für den Schnittpunkt bei pH 7 ergeben.

$$\text{Gesamtsäure:} \quad \frac{10 * \text{ml } 0,01 \text{ n NaOH}}{1 \text{ Träger}} = \text{μval/g}$$

Vorzugsweise ist der Pd/Au-Katalysator als Kugel ausgebildet. Entsprechend ist der Katalysatorträger als Kugel mit einem Durchmesser von bevorzugt größer als 1,5 mm, bevorzugter einem Durchmesser von größer als 3 mm und am bevorzugtesten mit einem Durchmesser von 4 mm bis 9 mm ausgebildet.

Zur Erhöhung der Aktivität des erfindungsgemäßen Pd/Au-Katalysators kann es vorgesehen sein, dass der Katalysatorträger mit zumindest einem Oxid eines Metalls dotiert ist, ausgewählt aus der Gruppe bestehend aus Zr, Hf, Ti, Nb, Ta, W, Mg, Re, Y und Fe, vorzugsweise mit $ZrO_2$, $HfO_2$ oder $Fe_2O_3$. Dabei kann es bevorzugt sein, wenn der Anteil des Katalysatorträgers an Dotierungsoxid zwischen 0 und 20 Mass.-% beträgt, vorzugsweise 1,0 bis 10 Mass.-% und bevorzugt 3 bis 8 Mass.-% bezogen auf die Masse des Katalysatorträgers.

Entsprechend einer alternativen Ausführungsform des erfindungsgemäßen Katalysators enthält dieser als katalytisch aktive Spezies Pd und Ag und um eine ausreichende Aktivität des Katalysators zu gewährleisten, vorzugsweise in der Hydrierung von Acetylen, beträgt der Anteil des Katalysators an Pd 0,01 bis 1,0 Mass.-%, vorzugsweise 0,02 bis 0,8 Mass.-% und bevorzugt 0,03 bis 0,7 Mass.-% bezogen auf die Masse des mit Edelmetall beladenen Katalysatorträgers.

Ebenfalls um eine ausreichende Aktivität des Katalysators in der Hydrierung von Acetylen zu erzielen, liegt das Ag/Pd-Atomverhältnis des Katalysators zwischen 0 und 10, vorzugsweise zwischen 1 und 5, wobei es bevorzugt ist, dass die Dicke der Edelmetallschale kleiner als 60 μm ist.

Entsprechend einer weiter bevorzugten Ausführungsform des erfindungsgemäßen Pd/Ag-Katalysators ist der Katalysatorträger als Kugel mit einem Durchmesser von größer als 1,5 mm ausgebildet, vorzugsweise mit einem Durchmesser von größer als 3 mm und bevorzugt mit einem Durchmesser von 2 bis 5 mm, oder als zylinderförmige Tablette mit Abmessungen von bis zu 7x7 mm.

Gemäß einer weiter bevorzugten Ausführungsform des erfindungsgemäßen Pd/Ag-Katalysators weist der Katalysatorträger eine Oberfläche von 1 bis 50 $m^2/g$ auf, vorzugsweise von zwischen 3 und 20 $m^2/g$.

Ferner kann es bevorzugt sein, dass der Katalysatorträger eine Oberfläche von kleiner/gleich 10 $m^2/g$ aufweist, vorzugsweise von kleiner als 5 $m^2/g$ und bevorzugt von kleiner als 2 $m^2/g$.

Ein bevorzugter erfindungsgemäßer Oxidations- oder Hydrierungskatalysator enthält als katalytisch aktive Spezies Pd und Pt, wobei der Anteil des Katalysators an Pd zur Gewährleistung einer ausreichenden Aktivität 0,05 bis 5 Mass.-% beträgt, vorzugsweise 0,1 bis 2,5 Mass.-% und bevorzugt 0,15 bis 0,8 Mass.-% bezogen auf die Masse des mit Edelmetall beladenen Katalysatorträgers.

Gemäß einer bevorzugten Ausführungsform des erfindungsgemäßen Pd/Pt-Katalysators liegt das Pd/Pt-Atomverhältnis des Katalysators zwischen 10 und 1, vorzugsweise zwischen 8 und 5 und bevorzugt zwischen 7 und 4.

Entsprechend einer weiter bevorzugten Ausführungsform des erfindungsgemäßen Pd/Pt-Katalysators ist der Katalysatorträger als Zylinder ausgebildet, vorzugsweise mit einem Durchmesser von 0,75 bis 3 mm und mit einer Länge von 0,3 bis 7 mm.

Ferner kann es bevorzugt sein, dass der Katalysatorträger eine Oberfläche von 50 bis 400 $m^2/g$ aufweist, vorzugsweise eine von zwischen 100 und 300 $m^2/g$.

Auch kann es bevorzugt sein, dass der Katalysator als katalytisch aktive Spezies metallisches Co, Ni und/oder Cu in der Schale enthält.

Entsprechend einer weiter bevorzugten Ausführungsform des erfindungsgemäßen Katalysators ist vorgesehen, dass der Katalysatorträger ein Träger auf der Basis eines Siliziumoxids, eines Aluminiumoxids, eines Alumosilikats, eines Zirkoniumoxids, eines Titanoxids, eines Nioboxids oder eines natürlichen Schichtsilikats, vorzugsweise eines kalzinierten säurebehandelten Bentonits, ist.

Wie bereits vorstehend ausgeführt, unterliegt der Katalysatorträger des erfindungsgemäßen Katalysators bei der Katalysatorherstellung einer gewissen mechanischen Belastung. Darüber hinaus kann der erfindungsgemäße Katalysator bei der Befüllung eines Reaktors mechanisch stark beansprucht werden, wodurch es zu einer unerwünschten Staubentwicklung sowie einer Beschädigung des Katalysatorträgers, insbesondere seiner in einem äußeren Bereich gelegenen, katalytisch aktiven Schale kommen kann. Insbesondere um den Abrieb des erfindungsgemäßen Katalysators in vertretbaren Grenzen zu halten, weist der Katalysatorträger eine Härte von größer/gleich 20 N auf, vorzugsweise eine von größer/gleich 30 N, weiter bevorzugt eine von größer/gleich 40 N und am meisten bevorzugt eine von größer/gleich 50 N. Die Druckhärte ist dabei wie vorstehend beschrieben bestimmt.

Der erfindungsgemäße Katalysator kann als Katalysatorträger vorzugsweise einen Katalysatorträger auf der Basis eines natürlichen Schichtsilikats, insbesondere eines kalzinierten säurebehandelten Bentonits, umfassen. Der Ausdruck "auf der Basis" bedeutet dabei im Rahmen der vorliegenden Erfindung, dass der Katalysator das entsprechende Material umfasst.

Erfindungsgemäß ist es bevorzugt, wenn der Anteil des Katalysatorträgers an dem Bentonit größer/gleich 50 Mass.-% ist, vorzugsweise größer/gleich 60 Mass.-%, bevorzugt größer/gleich 70 Mass.-%, weiter bevorzugt größer/gleich 80 Mass.-%, mehr bevorzugt größer/gleich 90 Mass.-% und am meisten bevorzugt größer/gleich 95 Mass.-% bezogen auf die Masse des Katalysatorträgers.

Es konnte festgestellt werden, dass die Produktselektivität des erfindungsgemäßen Pd/Au-Katalysators umso höher ist, je größer das integrale Porenvolumen des Katalysatorträgers ist. Gemäß einer weiter bevorzugten Ausführungsform des erfindungsgemäßen Katalysators weist daher der Katalysatorträger ein integrales Porenvolumen nach BJH von größer als 0,30 ml/g auf, vorzugsweise eines von größer als 0,35 ml/g und bevorzugt eines von größer als 0,40 ml/g.

Ferner kann es bevorzugt sein, wenn der Katalysatorträger des Pd/Au-Katalysators ein integrales Porenvolumen nach BJH von zwischen 0,3 und 1,2 ml/g aufweist, vorzugsweise eines von zwischen 0,4 und 1,1 ml/g und bevorzugt eines von 0,5 bis 1,0 ml/g.

Dabei ist das integrale Porenvolumen des Katalysatorträgers nach der Methode von BJH mittels Stickstoffadsorption bestimmt. Die Oberfläche des Katalysatorträgers sowie sein integrales Porenvolumen werden nach der BET- bzw. nach der BJH-Methode bestimmt. Die Bestimmung der BET-Oberfläche erfolgt nach der BET-Methode gemäß DIN 66131; eine Veröffentlichung der BET-Methode findet sich auch in J. Am. Chem. Soc. 60, 309 (1938). Zur Bestimmung der Oberfläche und des integralen Porenvolumens des Katalysatorträgers oder des Katalysators kann die Probe beispielsweise mit einem vollautomatischen Stickstoffporosimeter der Firma Mikromeritics, Typ ASAP 2010 vermessen werden, mittels dessen eine Adsorptions- sowie Desorptionsisotherme aufgenommen wird.

Zur Ermittlung der Oberfläche und der Porosität des Katalysatorträgers oder des Katalysators nach der BET-Theorie werden die Daten gemäß DIN 66131 ausgewertet. Das Porenvolumen wird aus den Messdaten unter Anwendung der BJH-Methode ermittelt (E.P. Barret, L.G. Joiner, P.P. Haienda, J. Am. Chem. Soc. (73/1951, 373)). Bei diesem Verfahren werden auch Effekte der Kapillarkondensation berücksichtigt. Porenvolumina bestimmter Porengrößenbereiche werden durch Aufsummieren inkrementeller Porenvolumina bestimmt, die aus der Auswertung der Adsorptionsisotherme nach BJH erhalten werden. Das integrale Porenvolumen nach der BJH-Methode bezieht sich auf Poren mit einem Durchmesser von 1,7 bis 300 nm.

Gemäß einer weiter bevorzugten Ausführungsform des erfindungsgemäßen Katalysators kann es vorgesehen sein, wenn die Wassersaugfähigkeit des Katalysatorträgers 40 bis 75 % beträgt, bevorzugt 50 bis 70 % berechnet als Gewichtszunahme durch Wasseraufnahme. Die Saugfähigkeit wird bestimmt, indem 10 g der Trägerprobe mit entionisiertem Wasser 30 min lang getränkt wird, bis von der Trägerprobe keine Gasblasen mehr entweichen. Dann wird das überschüssige Wasser dekantiert und die getränkte Probe mit einem Baumwolltuch abgetupft zur Befreiung der Probe von anhaftender Feuchtigkeit. Anschließend wird der wasserbeladene Träger ausgewogen und die Saugfähigkeit berechnet gemäß:

$$(\text{Auswaage (g)} - \text{Einwaage (g)}) \times 10 = \text{Wassersaugfähigkeit (\%)}$$

Gemäß einer weiter bevorzugten Ausführungsform des erfindungsgemäßen Katalysators kann es bevorzugt sein, wenn zumindest 80 % des integralen Porenvolumens des Katalysatorträgers von Mesoporen und Makroporen gebildet sind, vorzugsweise zumindest 85 % und bevorzugt zumindest 90 %. Dadurch wird einer, durch Diffusionslimitierung bewirkten, verminderten Aktivität des erfindungsgemäßen Katalysators entgegengewirkt, insbesondere bei Schalen mit verhältnismäßig großen Dicken. Dabei sollen diesbezüglich unter den Begriffen Mikroporen, Mesoporen und Makroporen Poren verstanden werden, die einen Durchmesser von kleiner als 2 nm, einen Durchmesser von 2 bis 50 nm bzw. einen Durchmesser von größer als 50 nm aufweisen.

Der Katalysatorträger des erfindungsgemäßen Katalysators ist als Formkörper ausgebildet. Dabei kann der Katalysatorträger grundsätzlich die Form eines jeglichen geometrischen Körpers annehmen, auf dem sich eine entsprechende Schale aufbringen lässt. Bevorzugt ist es jedoch, wenn der Katalysatorträger als Kugel, Zylinder (auch mit abgerundeten Stirnflächen), Lochzylinder (auch mit abgerundeten Stirnflächen), Trilobus, "capped tablet", Tetralobus, Ring, Donut, Stern, Wagenrad, "inverses" Wagenrad, oder als Strang, vorzugsweise als Rippstrang oder Sternstrang, ausgebildet ist.

Der Durchmesser bzw. die Länge und Dicke des Katalysatorträgers des erfindungsgemäßen Katalysators beträgt vorzugsweise 1 bis 9 mm, je nach Reaktorrohrgeometrie, in dem der Katalysator Einsatz finden soll.

Die Produktselektivität des erfindungsgemäßen Katalysators ist im Allgemeinen umso höher, je geringer die Dicke der Schale des Katalysators ist. Entsprechend einer weiter bevorzugten Ausführungsform des erfindungsgemäßen Katalysators weist daher die Schale des Katalysators eine Dicke von kleiner als 300 μm auf, vorzugsweise eine von kleiner als 200 μm, bevorzugt eine von kleiner als 150 μm, weiter bevorzugt eine von kleiner als 100 μm und mehr bevorzugt eine von kleiner als 80 μm. Die Dicke der Schale kann im Falle von geträgerten Metallkatalysatoren häufig mittels eines Mikroskops optisch ausgemessen werden. Und zwar erscheint der Bereich, in dem die Metalle abgeschieden sind, schwarz, während die metallfreien Bereiche weiß erscheinen. Die Grenzlinie zwischen metallhaltigen und - freien Bereichen ist in der Regel sehr scharf und optisch deutlich zu erkennen. Sollte die vorgenannte Grenzlinie nicht scharf ausgebildet und entsprechend optisch nicht deutlich zu erkennen sein oder die Schalendicke aus sonstigen Gründen optisch nicht bestimmbar sein, so entspricht die Dicke der Schale der Dicke einer Schale, gemessen ausgehend von der äußeren Oberfläche des Katalysatorträgers, in welcher 95 % der auf dem Träger abgeschiedenen katalytisch aktiven Spezies enthalten sind.

Es konnte ebenfalls festgestellt werden, dass bei dem erfindungsgemäßen Katalysator die Schale mit einer eine hohe Aktivität des Katalysators bewirkenden, verhältnismäßig großen Dicke ausgebildet werden kann, ohne eine nennenswerte Verminderung der Produktselektivität des erfindungsgemäßen Katalysators zu bewirken. Hiezu sind Katalysatorträger mit einer verhältnismäßig geringen Oberfläche einzusetzen. Entsprechend einer anderen bevorzugten Ausführungsform des erfindungsgemäßen Katalysators weist die Schale des Katalysators daher eine Dicke von zwischen 200 und 2000 μm auf, vorzugsweise eine von zwischen 250 und 1800 μm, bevorzugt eine von zwischen 300 und 1500 μm und weiter bevorzugt eine von zwischen 400 und 1200 μm.

Die vorliegende Erfindung betrifft ferner die Verwendung einer Vorrichtung, die eingerichtet ist, mittels eines Prozessgases ein Fließbett von Katalysatorträger-Formkörpern zu erzeugen, in welchem die Katalysatorträger-Formkörper elliptisch oder toroidal umlaufen, vorzugsweise toroidal, zur Durchführung des erfindungsgemäßen Verfahrens oder bei der Herstellung des erfindungsgemäßen Schalenkatalysators. Es wurde festgestellt, dass sich mittels derartiger Vorrichtungen Schalenkatalysatoren herstellen lassen, welche die vorgenannten vorteilhaften Eigenschaften aufweisen.

Entsprechend einer bevorzugten Ausführungsform der erfindungsgemäßen Verwendung ist es vorgesehen, dass die Vorrichtung eine Prozesskammer mit einem Boden und einer Seitenwand umfasst, wobei der Boden aus mehreren, übereinander gelegten, sich einander überlappenden, ringförmigen Leitplatten aufgebaut ist, zwischen denen ringförmige Schlitze ausgebildet sind, über die Prozessgas mit einer horizontalen, radial nach außen gerichteten Bewegungskomponente einführbar ist. Dadurch wird auf eine verfahrenstechnisch einfache Weise die Ausbildung eines Fließbettes ermöglicht, in welcher die Formkörper besonders gleichmäßig elliptisch umlaufen, was mit einer Steigerung der Produktqualität einhergeht. Diese elliptische Umlaufbewegung kann mittels einer zweiten Bewegungskomponente, wie sie beispielsweise entweder durch an den ringförmigen Schlitzen angebrachte Leitelemente oder einer weiteren vertikalen Prozessgaskomponente erzeugt werden kann, in ein Fließbett umgewandelt werden, das ein toroidales Umlaufen der Formkörper ermöglicht.

Um ein besonders gleichmäßiges Besprühen der Formkörper beispielsweise mit Edelmetalllösungen zu gewährleisten, kann gemäß einer weiteren Ausführungsform vorgesehen sein, dass mittig im Boden eine Ringspaltdüse angeordnet ist, deren Mündung derart ausgebildet ist, dass mit der Düse eine Sprühwolke versprühbar ist, deren Spiegelebene parallel zur Bodenebene verläuft.

Ferner kann es bevorzugt sein, dass zwischen der Mündung der Ringspaltdüse und dem darunterliegenden Boden Austrittsöffnungen für Stützgas vorgesehen sind, um an der Unterseite der Sprühwolke ein Stützpolster zu bewerkstelligen. Das bodenseitige Luftkissen hält die Bodenoberfläche frei von versprühter Lösung, das heißt, dass die gesamte versprühte Lösung in das Fließbett der Formkörper eingetragen wird, so dass keine Sprühverluste auftreten, was insbesondere hinsichtlich teurer Edelmetallverbindungen oder Enzyme aus Kostengründen von Bedeutung ist.

Entsprechend einer weiter bevorzugten Ausführungsform der erfindungsgemäßen Verwendung wird in der Vorrichtung das Stützgas von der Ringspaltdüse selbst und/oder durch Prozessgas bereitgestellt. Diese Maßnahmen lassen sehr variable Ausgestaltungen der Bewerkstelligung des Stützgases zu. Es können an der Ringspaltdüse selbst Austrittsöffnungen vorgesehen sein, über die ein Teil des Sprühgases austritt, um zur Ausbildung des Stützgases beizutragen. Zusätzlich oder alternativ können Teile des Prozessgases, das durch den Boden strömt, in Richtung der Unterseite der Sprühwolke geführt werden und dadurch zur Ausbildung des Stützgases beitragen.

Entsprechend einer weiteren Ausführungsform der Erfindung weist die Ringspaltdüse einen kegelförmigen Kopf auf und die Mündung verläuft entlang einer kreisförmigen Kegelschnittfläche. Dadurch wird gewährleistet, dass durch den Kegel die sich vertikal von oben nach unten bewegenden Formkörper gleichmäßig und gezielt auf die Sprühwolke zugeführt werden, die vom kreisförmigen Sprühspalt im unteren Ende des Kegels versprüht wird.

Entsprechend einer weiteren Ausführungsform der Verwendung ist im Bereich zwischen Mündung und darunterliegendem Boden eine kegelstumpfförmige Wand vorgesehen, die vorzugsweise Durchtrittsöffnungen für Stützgas aufweist. Diese Maßnahme hat den Vorteil, dass die zuvor erwähnte harmonische Umlenkbewegung am Kegel durch die Fortsetzung über den Kegelstumpf aufrechterhalten wird und in diesem Bereich Stützgas durch die Durchtrittsöffnungen austreten kann und für die entsprechende Stützung an der Unterseite der Sprühwolke sorgt.

In einer weiteren Ausgestaltung der Verwendung ist zwischen der Unterseite der kegelstumpfförmigen Wand ein ringförmiger Schlitz zum Durchtritt von Prozessgas ausgebildet. Diese Maßnahme hat den Vorteil, dass der Übergang der Formkörper auf das Luftpolster des Bodens besonders gut gesteuert werden kann und unmittelbar im Bereich unter der Düse beginnend gezielt durchgeführt werden kann.

Um die Sprühwolke in gewünschter Höhe in das Fließbett eintragen zu können, ist es bevorzugt, dass die Lage der Mündung der Düse in der Höhe verstellbar ist.

Entsprechend einer weiteren Ausgestaltung der erfindungsgemäßen Verwendung sind zwischen den ringförmigen Leitplatten Leitelemente angeordnet, die dem durchtretenden Prozessgas eine umfängliche Strömungskomponente auferlegen.

Ausführungsbeispiele

Die nachstehenden Ausführungsbeispiele dienen der Erläuterung der Erfindung.

Beispiel 1:

225 g kugelförmige, aus einem säurebehandelten kalzinierten Bentonit als natürliches Schichtsilikat gebildete Katalysatorträger-Formkörper der Firma SÜD-Chemie AG (München, Deutschland) mit der Handelsbezeichnung "KA-0" und den in der Tabelle 1 aufgeführten Charakteristika:

Tabelle 1:

| Geometrische Form | Kugel |
|---|---|
| Durchmesser | 5 mm |
| Feuchtegehalt | 2,5 Mass.-% |

(fortgesetzt)

| | |
|---|---|
| Druckfestigkeit | 52 N |
| Schüttgewicht | 528 g l$^{-1}$ |
| Wassersaugvermögen | 69,5 % |
| spezif. Oberfläche (BET) | 104 m$^2$ g$^{-1}$ |
| SiO$_2$-Gehalt | ≥95 Mass.-% |
| Al$_2$O$_3$-Gehalt | 1,0 Mass.-% |
| Fe$_2$O$_3$-Gehalt | 0,2 Mass.-% |
| TiO$_2$-Gehalt | (Summe) < 1,5 Mass.-% |
| MgO-Gehalt | |
| CaO-Gehalt | |
| K$_2$O-Gehalt | |
| Na$_2$O-Gehalt | |
| Glühverlust 1000 °C | <0,3 Mass.-% |
| Azidität | 80 $\mu$val/g |
| BJH Porenvolumen N$_2$ | 0,4 cm$^3$ g$^{-1}$ |

wurden in einer Fließbettvorrichtung des Unternehmens Innojet Technologies (Lörrach, Deutschland) mit der Handelsbezeichnung Innojet® Aircoater gefüllt und mittels auf 100 °C temperierter Druckluft (6 bar) in einen Fließbettzustand versetzt, in welchem die Formkörper toroidal umliefen, d.h. sich auf einer vertikal ausgerichteten ellipsoiden und einer dazu senkrecht ausgerichteten horizontalen Kreisbahn bewegten.

Nachdem die Formkörper auf eine Temperatur von ca. 75 °C temperiert waren, wurden auf das Fließbett der Formkörper 300 ml einer wässrigen Edelmetallmischlösung enthaltend 7,5 g handelsübliches Na$_2$PdCl$_4$ (Natriumtetrachloropalladat) und 4,6 g handelsübliches NaAuCl$_4$ (Natriumtetrachloroaurat) über einen Zeitraum von 40 min aufgesprüht.

Nach der Imprägnierung der Katalysatorträger mit der Edelmetallmischlösung wurde auf das Fließbett der Formkörper eine 0,05 molare NaOH-Lösung bei einer Temperatur von 80 °C über einen Zeitraum von 30 min aufgesprüht. Dabei scheidet sich das NaOH überwiegend innerhalb der Schale ab und fixiert die Pd- und Au-Metallkomponenten, ohne dass der Träger allzu starken NaOH-Konzentrationen ausgesetzt wird.

Nach der NaOH-Einwirkung wurden die Träger ausgiebig in der Fließbettvorrichtung mit Wasser gewaschen, um den Träger weitestgehend von über die Edelmetallverbindungen und NaOH in den Träger eingebrachtem Alkalimetall und Chlorid zu befreien.

Nach dem Waschen wurden die Formkörper durch Bewegen in heißer Prozessluft (100 °C) in der Fließbettvorrichtung getrocknet.

Nach dem Trocknen der Formkörper wurden diese mit einem Gasgemisch von Ethylen (5 Vol.-%) in Stickstoff bei einer Temperatur von etwa 150 °C in der Fließbettvorrichtung zu einem Pd/Au-Schalenkatalysator reduziert.

Der resultierende Schalenkatalysator enthielt ca. 1,2 Mass.-% Pd und wies ein Au/Pd-Atomverhältnis von ca. 0,5, eine Schalendicke von ca. 160 $\mu$m sowie eine Härte von 38 N auf.

Die Edelmetallkonzentration des so hergestellten Pd/Au-Schalenkatalysators wich über einen Bereich von 90 % der Schalendicke hinweg, wobei der Bereich zur äußeren und inneren Schalengrenze jeweils um 5 % der Schalendicke beabstandet ist, von der mittleren Edelmetallkonzentration dieses Bereichs um maximal +/- 10 % ab.

Die Bestimmung der Edelmetallverteilung erfolgte an einem Rasterelektronenmikroskop LEO 430VP, ausgerüstet mit einem energiedispersiven Spektrometer der Fa. Bruker AXS. Zur Messung der Edelmetallkonzentration über die Schalendicke hinweg wurde eine Katalysatorkugel durchschnitten, auf einen Aluminiumprobenhalter geklebt und anschließend mit Kohlenstoff bedampft. Als Detektor kam ein stickstofffreier Siliziumdriftkammerdetektor (XFlash® 410) mit einer Energieauflösung von 125 eV für die Mangan K$_{alpha}$-Linie zum Einsatz.

Die folgenden Parameter wurden für die Messung verwendet:

Scanauflösung: 500 Punkte
Abstand der Messpunkte: 1,8 $\mu$m

Vergrößerung: 200 fach
Strahlspannung 20 kV
Strahlstrom 20 nA
Eingangsimpulsrate: 50000 Impulse/s
Messzeit für Linescan: 200 s

Für andere Elemente (siehe nachfolgende Beispiele) werden die entsprechenden verfügbaren Linien zur Messung herangezogen.

Von 100 Kugeln der wie vorstehend beschrieben hergestellten Schalenkatalysatorcharge wurde die Schalendicke vermessen.

Dabei war das Verhältnis der Standardabweichung der Schalendicken der Schalenkatalysatoren der Charge zum Mittelwert der Schalendicken der Schalenkatalysatoren der Charge kleiner als 10 %.

Beispiel 2:

Es wurden Schalenkatalysatoren analog Beispiel 1 hergestellt mit der Ausnahme, dass anstatt der Pd/Au-Lösung eine Lösung enthaltend 25 mmol $CuCl_2$ eingesetzt wurde und dass nicht fixiert und nicht gewaschen wurde.

Der resultierende Schalenkatalysator enthielt ca. 0,7 Mass.-% Cu und wies eine Schalendicke von ca. 136 $\mu$m auf.

Die Metallkonzentration des so hergestellten Schalenkatalysators wich über einen Bereich von 90 % der Schalendicke hinweg, wobei der Bereich zur äußeren und inneren Schalengrenze jeweils um 5 % der Schalendicke beabstandet ist, von der mittleren Metallkonzentration dieses Bereichs um maximal +/- 20 % ab (Messung wie in Beispiel 1).

Von 100 Kugeln der wie vorstehend beschrieben hergestellten Schalenkatalysatorcharge wurde die Schalendicke vermessen. Dabei war das Verhältnis der Standardabweichung der Schalendicken der Schalenkatalysatoren der Charge zum Mittelwert der Schalendicken der Schalenkatalysatoren der Charge kleiner als 10 %.

Beispiel 3:

Es wurden Schalenkatalysatoren analog Beispiel 1 hergestellt mit der Ausnahme, dass anstatt der Pd/Au-Lösung eine Lösung enthaltend 26 mmol $Na_2PdCl_4$ eingesetzt wurde und weder fixiert noch gewaschen wurde.

Der resultierende Schalenkatalysator enthielt ca. 1,0 Mass.-% Pd und wies eine Schalendicke von ca. 93 $\mu$m auf.

Die Metallkonzentration des so hergestellten Schalenkatalysators wich über einen Bereich von 90 % der Schalendicke hinweg, wobei der Bereich zur äußeren und inneren Schalengrenze jeweils um 5 % der Schalendicke beabstandet ist, von der mittleren Metallkonzentration dieses Bereichs um maximal +/- 20 % ab (Messung wie in Beispiel 1).

Von 100 Kugeln der wie vorstehend beschrieben hergestellten Schalenkatalysatorcharge wurde die Schalendicke vermessen. Dabei war das Verhältnis der Standardabweichung der Schalendicken der Schalenkatalysatoren der Charge zum Mittelwert der Schalendicken der Schalenkatalysatoren der Charge kleiner als 10 %.

Beispiel 4:

Es wurden Schalenkatalysatoren analog Beispiel 1 hergestellt mit der Ausnahme, dass anstatt der Pd/Au-Lösung eine Lösung enthaltend 26 mmol $Pd(NH_3)_4(OH)_2$ eingesetzt wurde und weder fixiert noch gewaschen wurde.

Der resultierende Schalenkatalysator enthielt ca. 1,0 Mass.-% Pd und wies eine Schalendicke von ca. 71 $\mu$m auf.

Die Metallkonzentration des so hergestellten Schalenkatalysators wich über einen Bereich von 90 % der Schalendicke hinweg, wobei der Bereich zur äußeren und inneren Schalengrenze jeweils um 5 % der Schalendicke beabstandet ist, von der mittleren Metallkonzentration dieses Bereichs um maximal +/- 20 % ab (Messung wie in Beispiel 1).

Von 100 Kugeln der wie vorstehend beschrieben hergestellten Schalenkatalysatorcharge wurde die Schalendicke vermessen. Dabei war das Verhältnis der Standardabweichung der Schalendicken der Schalenkatalysatoren der Charge zum Mittelwert der Schalendicken der Schalenkatalysatoren der Charge kleiner als 10 %.

Vergleichsbeispiel 1:

65,02 g Katalysatorträger-Formkörpern der Firma Süd-Chemie AG (München, Deutschland) mit der Handelsbezeichnung "KA-160" mit den in Tabelle 2 angegebenen Kenndaten werden gemäß dem Porenfüllverfahren (incipient-wetness-Methode), bei welchem ein Träger mit einem seinem Porenvolumen entsprechenden Lösungsvolumen imprägniert wird, mit 39,1 ml einer wässrigen Lösung beinhaltend 1,568 g $Na_2PdCl_4$ und 0,367 g $HAuCl_4$ imprägniert. Nach der Imprägnierung werden 89,17 g einer 0,35 molaren NaOH-Lösung auf die Katalysatorträger-Formkörper gegeben und über Nacht bei Raumtemperatur für 22 Stunden stehen gelassen. Nach Dekantieren der Fixierlösung wird der so hergestellte Katalysatorvorläufer mit 73,68 g einer 10 %-igen $NaH_2PO_2$-Lösung (Fluka) 2 Stunden lang reduziert. Nach Ablassen der Reduktionslösung werden die Katalysatoren mit dest. Wasser 8 Stunden bei Raumtemperatur unter ständigem Austausch des Wassers (Durchfluss = 140 rpm) zur Entfernung von Cl-Resten gewaschen. Der Endwert der Leitfähigkeit der Waschlösung beträgt 1,2 $\mu$S.

Im Anschluss wird der Katalysator in der Wirbelschicht bei 90 °C für 50 min getrocknet. Die getrockneten Kugeln werden mit einer Mischung aus 27,29 g 2 molarer KOAc-Lösung und 18,55 g $H_2O$ beladen und eine Stunde bei Raumtemperatur stehen gelassen. Zum Abschluss erfolgt die Trocknung für 40 min bei 90 °C in der Wirbelschicht.

Die theoretische Metallbeladung beträgt 0,8 Gew.-% Pd und 0,3 Gew.-% Au; die durch Elementaranalyse mittels ICP (Inductively Coupled Plasma) experimentell bestimmten Werte betrugen 0,78 Gew.-% Pd und 0,26 Gew.-% Au.

Die Schalendicke betrug 280 $\mu$m.

Tabelle 2:

| Geometrische Form | Kugel |
|---|---|
| Durchmesser | 5 mm |
| Feuchtegehalt | <2,0 Mass.-% |
| Druckfestigkeit | > 60 N |
| Schüttgewicht | 554 g l$^{-1}$ |
| Wassersaugvermögen | 62 % |
| spezif. Oberfläche (BET) | 158 m$^2$ g$^{-1}$ |
| $SiO_2$-Gehalt | 93,2 Mass.-% |
| $Al_2O_3$-Gehalt | 2,2 Mass.-% |
| $Fe_2O_3$-Gehalt | 0,35 Mass.-% |
| $TiO_2$-Gehalt | |
| MgO-Gehalt | |
| CaO-Gehalt | (Summe) < 1,5 Mass.-% |
| $K_2O$-Gehalt | |
| $Na_2O$-Gehalt | |
| Glühverlust 1000 °C | <0,3 Mass.-% |
| Azidität | 53 $\mu$val/g |
| BJH Porenvolumen $N_2$ | 0,38 cm$^3$ g$^{-1}$ |

Beispiel 5:

65,02 g kugelförmige, aus einem säurebehandelten kalzinierten Bentonit als natürliches Schichtsilikat gebildete Katalysatorträger-Formkörper der Firma SÜD-Chemie AG (München, Deutschland) mit der Handelsbezeichnung "KA-160" und den in der Tabelle 2 aufgeführten Charakteristika wurden in einer Fließbettvorrichtung des Unternehmens Innojet Technologies (Lörrach, Deutschland) mit der Handelsbezeichnung Innojet® Aircoater gefüllt und mittels auf 90 °C temperierter Druckluft (6 bar) in einen Fließbettzustand versetzt, in welchem die Formkörper toroidal umliefen.

Auf das Fließbett der Formkörper wurden 300 ml einer wässrigen Lösung beinhaltend 1,568 g $Na_2PdCl_4$ und 0,367 g $HAuCl_4$ über einen Zeitraum von 40 min aufgesprüht.

Nach der Imprägnierung werden 89,17 g einer 0,35 molaren NaOH-Lösung auf die Katalysatorträger-Formkörper gegeben und über Nacht bei Raumtemperatur für 22 Stunden stehen gelassen. Nach Dekantieren der Fixierlösung wird der so hergestellte Katalysatorvorläufer mit 73,68 g einer 10 %-igen $NaH_2PO_2$-Lösung (Fluka) 2 Stunden lang reduziert. Nach Ablassen der Reduktionslösung werden die Katalysatoren mit dest. Wasser 8 Stunden bei Raumtemperatur unter ständigem Austausch des Wassers (Durchfluss = 140 rpm) zur Entfernung von Cl-Resten gewaschen. Der Endwert der Leitfähigkeit der Waschlösung beträgt 1,2 $\mu$S.

Im Anschluss wird der Katalysator in der Wirbelschicht bei 90 °C für 50 min getrocknet. Die getrockneten Kugeln werden mit einer Mischung aus 27,29 g 2 molarer KOAc-Lösung und 18,55 g $H_2O$ beladen und eine Stunde bei Raumtemperatur stehen gelassen.

Zum Abschluss erfolgt die Trocknung für 40 min bei 90 °C in der Wirbelschicht.

Die theoretische Metallbeladung beträgt 0,8 Gew.-% Pd und 0,3 Gew.-% Au; die mittels ICP experimentell bestimmten Werte betrugen 0,75 Gew.-% Pd und 0,25 Gew.-% Au.

Die Schalendicke betrug 205 $\mu$m.

Beispiel 6:

Reaktortest

6 ml einer Schüttung von Katalysatorkugeln gemäß Beispiel 5 und des Vergleichsbeispiels 1 wurden jeweils in einem Festbettröhrenreaktor bei einer Temperatur von 150 °C bei 10 bar mit einem Feedgasstrom von 550 Nml/min zusammengesetzt aus 15 % HOAc, 6 % $O_2$, 39 % $C_2H_4$ in $N_2$ beaufschlagt und der Reaktoraustrag mittels Gaschromatographie analysiert.

Die Selektivität (von Ethylen zu VAM) wird nach der Formel $S(C_2H_4)$ = mole VAM / (mole VAM + mole $CO_2$/2) berechnet. Die Raum-Zeit-Ausbeute ergibt sich als g VAM/l Katalysator/h. Der Sauerstoff-Umsatz wird berechnet nach (mole $O_2$ in - mole $O_2$ out)/mole $O_2$ in.

Der mittels des erfindungsgemäßen Verfahrens gemäß Beispiel 5 hergestellte Katalysator zeigt eine Selektivität $S(C_2H_4)$ von 92,3 % sowie eine Raum-Zeit-Ausbeute (gaschromatographisch bestimmt) von 654 g VAM / l Katalysator / h bei einem Sauerstoffumsatz von 38,7 %.

Der Katalysator gemäß Vergleichsbeispiel 1 zeigte eine Selektivität $S(C_2H_4)$ von 91,0 % sowie eine Raum-Zeit-Ausbeute (gaschromatographisch bestimmt) von 576 g VAM / 1 Katalysator / h bei einem Sauerstoffumsatz von 36,1 %.

Der mittels des erfindungsgemäßen Verfahrens hergestellte Katalysator gemäß Beispiel 5 zeigt sowohl eine höhere Selektivität als auch Aktivität in der VAM-Synthese im Vergleich zu einem Katalysator hergestellt nach einem Verfahren des Standes der Technik gemäß Vergleichsbeispiel 1.

Zeichnung

Die nachstehende Beschreibung einer bevorzugten Vorrichtung zur Durchführung des erfindungsgemäßen Verfahrens sowie die Beschreibung von Bewegungsbahnen von Katalysatorträger-Formkörpern dient im Zusammenhang mit der Zeichnung der Erläuterung der Erfindung. Es zeigen:

Fig. 1A    eine vertikale Schnittansicht einer bevorzugten Vorrichtung zur Durchführung des erfindungsgemäßen Verfahrens;

Fig. 1B    eine Vergrößerung des in der Fig. 1A umrahmten und mit dem Bezugszeichen 1B markierten Bereichs;

Fig. 2A    eine perspektivische Schnittansicht der bevorzugten Vorrichtung, in welcher die Bewegungsbahnen zweier elliptisch umlaufender Katalysatorträger-Formkörper schematisch dargestellt sind;

Fig. 2B    eine Draufsicht auf die bevorzugte Vorrichtung und die Bewegungsbahnen gemäß Fig. 2A;

Fig. 3A    eine perspektivische Schnittansicht der bevorzugten Vorrichtung, in welcher die Bewegungsbahn eines toroidal umlaufenden Katalysatorträger-Formkörpers schematisch dargestellt ist;

Fig. 3B    eine Draufsicht auf die bevorzugte Vorrichtung und die Bewegungsbahn gemäß Fig. 3A.

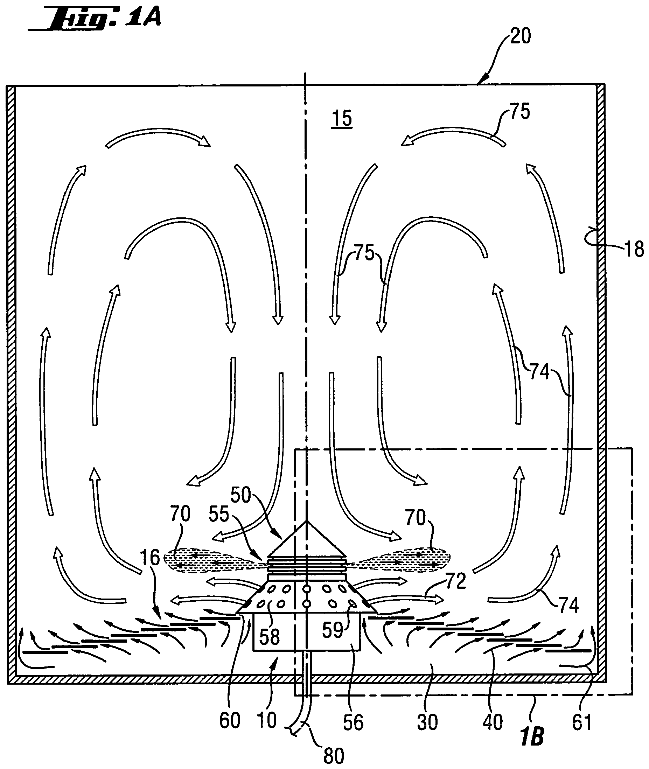

In der Fig. 1A ist eine insgesamt mit dem Bezugszeichen 10 belegte Vorrichtung zur Durchführung des erfindungsgemäßen Verfahrens gezeigt.

Die Vorrichtung 10 weist einen Behälter 20 mit einer aufrecht stehenden zylindrischen Seitenwand 18 auf, die eine Prozesskammer 15 umschließt.

Die Prozesskammer 15 weist einen Boden 16 auf, unter dem sich eine Anströmkammer 30 befindet.

Der Boden 16 ist aus insgesamt sieben ringförmigen übereinander gelegenen Ringplatten als Leitplatten zusammengesetzt. Die sieben Ringplatten sind so übereinander gesetzt, dass eine äußerste Ringplatte 25 eine unterste Ringplatte bildet, auf der dann die weiteren sechs inneren Ringplatten, die jeweils darunter liegend, teilweise überlappend, aufgelegt sind.

Der Übersicht halber sind nur einige der insgesamt sieben Ringplatten mit Bezugszeichen versehen, beispielsweise die beiden übereinander liegenden Ringplatten 26 und 27. Durch diese Übereinanderlegung und Beabstandung ist zwischen zwei Ringplatten jeweils ein ringförmiger Schlitz 28 ausgebildet, durch den Prozessluft 40 als Prozessgas mit einer überwiegend horizontal gerichteten Bewegungskomponente durch den Boden 16 hindurch treten kann.

In der mittigen obersten inneren Ringplatte 29 ist in deren zentraler Öffnung von unten eine Ringspaltdüse 50 eingesetzt. Die Ringspaltdüse 50 weist eine Mündung 55 auf, die insgesamt drei Mündungsspalte 52, 53 und 54 aufweist. Alle drei Mündungsspalte 52, 53 und 54 sind so ausgerichtet, dass sie etwa parallel zum Boden 16, also etwa horizontal mit einem Umfassungswinkel von 360° aussprühen. Über den oberen Spalt 52 sowie den unteren Spalt 54 wird Sprühluft als Sprühgas ausgepresst, durch den mittleren Spalt 53 die zu versprühende Lösung.

Die Ringspaltdüse 50 weist einen stabförmigen Körper 56 auf, der nach unten fortreicht und die entsprechenden Kanäle und Zuführleitungen enthält, die an sich bekannt und deshalb in der Zeichnung nicht dargestellt sind. Die Ringspaltdüse 50 kann beispielsweise mit einem so genannten rotativen Ringspalt ausgebildet sein, bei dem sich Wände des Kanals, durch den die Lösung ausgesprüht wird, relativ zueinander drehen, um Verstopfungen der Düse zu vermeiden, so dass über den Umfassungswinkel von 360° gleichmäßig aus dem Spalt 53 ausgesprüht werden kann.

Die Ringspaltdüse 50 weist oberhalb des Mündungsspalts 52 einen kegelförmigen Kopf 57 auf.

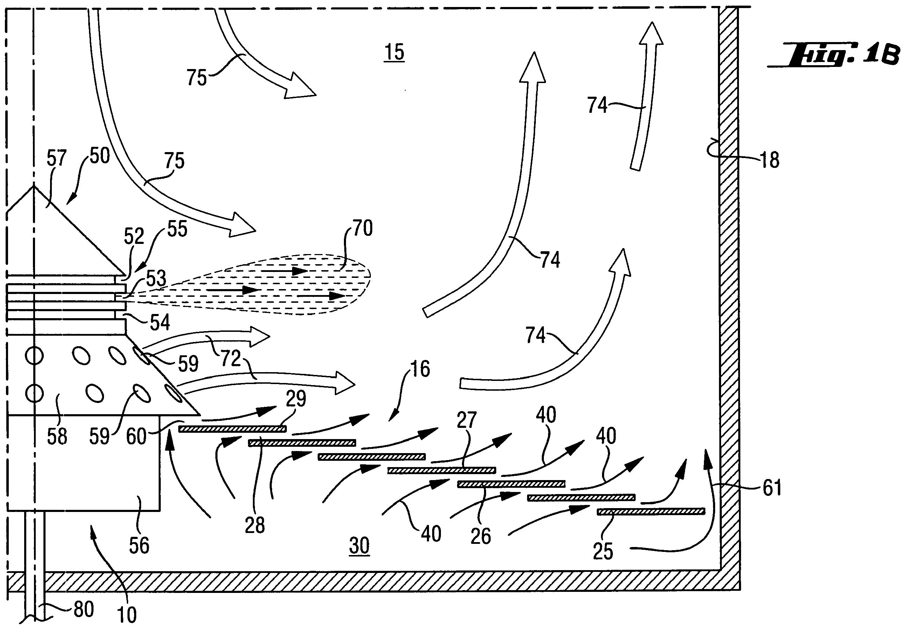

Im Bereich unterhalb des Mündungsspalts 54 ist eine kegelstumpfförmige Wand 58 vorhanden, die zahlreiche Öffnungen 59 aufweist. Wie aus der Fig. 1B zu erkennen ist, ruht die Unterseite der kegelstumpfförmigen Wand 58 auf der innersten Ringplatte 29 derart auf, dass zwischen der Unterseite der kegelstumpfförmigen Wand 58 und der darunter liegenden, mit dieser teilweise überlappenden Ringplatte 29 ein Schlitz 60 ausgebildet ist, durch den Prozessluft 40 hindurch treten kann.

Der äußere Ring 25 ist zu der Wand 18 beabstandet, so dass Prozessluft 40 in Richtung des mit dem Bezugszeichen 61 belegten Pfeiles mit einer überwiegend vertikalen Komponente in die Prozesskammer 15 eintreten kann und dadurch der durch die Schlitze 28 in die Prozesskammer 15 eintretenden Prozessluft 40 eine verhältnismäßig stark nach oben gerichtete Komponente verleiht.

Auf der rechten Hälfte der Fig. 1A ist dargstellt, welche Verhältnisse sich in einem eingelaufenen Zustand in der Vorrichtung 10 ausbilden.

Aus dem Mündungsspalt 53 tritt eine Sprühwolke 70 aus, deren horizontale Spiegelebene in etwa parallel zur Bodenebene verläuft. Durch die durch die Öffnungen 59 in der kegelstumpfförmigen Wand 58 durchtretende Luft, die beispielsweise Prozessluft 40 sein kann, bildet sich an der Unterseite der Sprühwolke 70 eine Stützluftströmung 72 aus. Durch die durch die zahlreichen Schlitze 28 hindurch tretende Prozessluft 40 bildet sich eine radiale Strömung in Richtung der Wand 18 aus, von der die Prozessluft 40 nach oben umgelenkt wird, wie es durch den mit dem Bezugszeichen 74 belegten Pfeil dargestellt ist. Von der umgelenkten Prozessluft 40 werden die Formkörper im Bereich der Wand 18 nach oben geführt. Die Prozessluft 40 und die zu behandelnden Katalysatorträger-Formkörper trennen sich dann voneinander, wobei die Prozessluft 40 durch Auslässe abgeführt wird, während sich die Formkörper radial gemäß der Pfeile 75 nach innen in Richtung des kegelförmigen Kopfes 57 der Ringspaltdüse 50 vertikal nach unten bewegen. Dort werden die Formkörper umgelenkt, auf die Oberseite der Sprühwolke 70 geleitet und dort mit dem versprühten Medium behandelt. Die besprühten Formkörper bewegen sich dann wieder in Richtung der Wand 18 und dabei voneinander weg, da nach Verlassen der Sprühwolke 70 an dem ringförmigen Mündungsspalt 53 den Formkörpern ein umfänglich größerer Raum zur Verfügung steht. Im Bereich der Sprühwolke 70 treffen die zu behandelnden Formkörper mit Flüssigkeitsteilchen zusammen und werden in Bewegungsrichtung in Richtung der Wand 18 bleibend voneinander weg bewegt und dabei sehr gleichmäßig und harmonisch mit der Prozessluft 40 behandelt und dabei getrocknet.

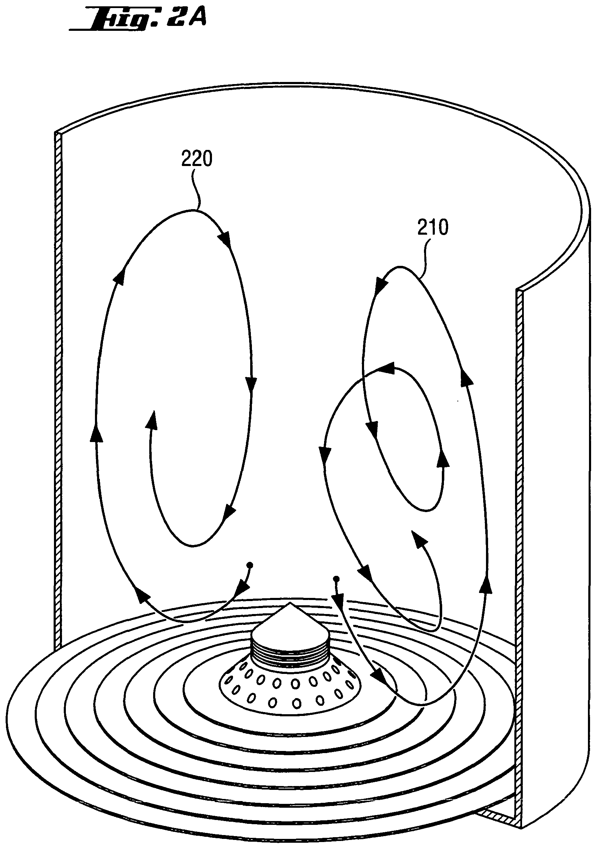

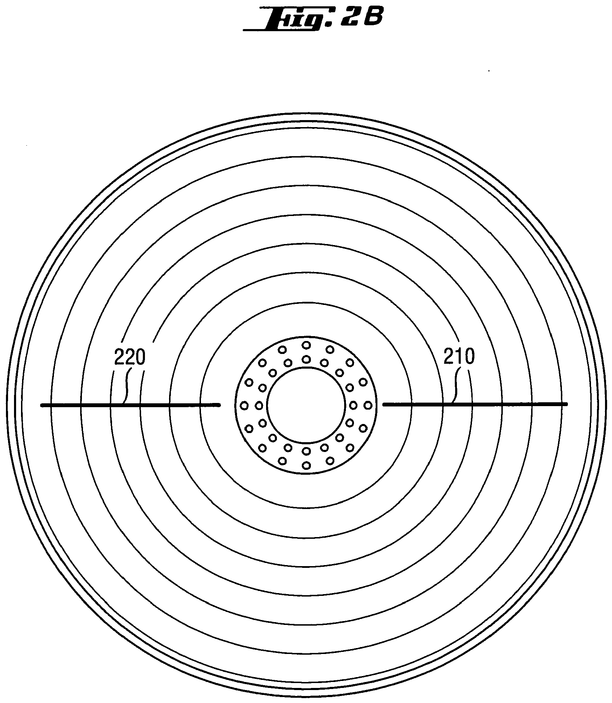

In der Figur 2A sind zwei mögliche Bewegungsbahnen zweier elliptisch umlaufender Katalysatorträger-Formkörper mittels der mit den Bezugszeichen 210 und 220 belegten Kurvenverläufe gezeigt. Die elliptische Bewegungsbahn 210 weist - im Vergleich zu einer idealen elliptischen Bahn - relativ große Änderungen in der Größe der Haupt- und Nebenachse auf. Die elliptische Bewegungsbahn 220 weist im Gegensatz dazu relativ kleine Änderung in der Größe der Haupt- und Nebenachse auf und beschreibt nahezu eine ideale elliptische Bahn ohne jegliche umfängliche (horizontale) Bewegungskomponente, wie aus der Figur 2B zu entnehmen ist.

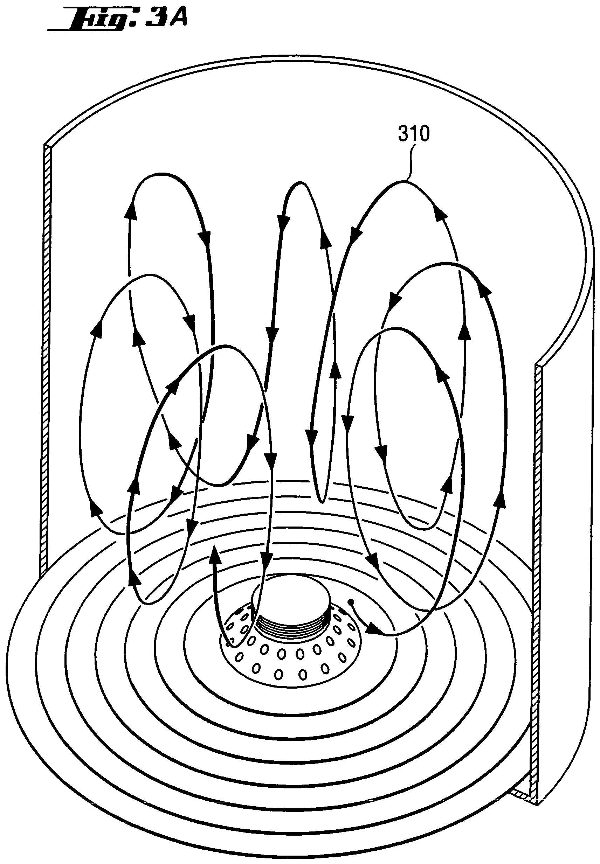

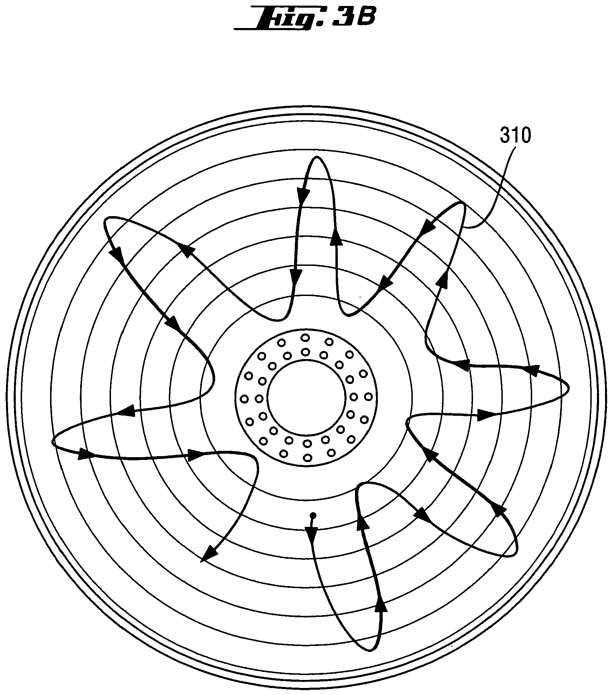

In der Figur 3A ist eine mögliche Bewegungsbahn eines toroidal umlaufenden Katalysatorträger-Formkörpers mittels des mit dem Bezugszeichen 310 belegten Kurvenverlaufs gezeigt. Die toroidal verlaufende Bewegungsbahn 310 beschreibt einen Ausschnitt der Oberfläche eines nahezu gleichförmigen Torus, dessen vertikaler Schnitt ellipsenförmig und dessen horizontaler Schnitt ringförmig ist. Die Figur 3B zeigt die Bewegungsbahn 310 in Draufsicht.

**Patentansprüche**

1. Verfahren zur Herstellung eines Schalenkatalysators, der einen porösen Katalysatorträger-Formkörper mit einer äußeren Schale umfasst, in welcher zumindest eine katalytisch aktive Spezies enthalten ist, wobei das Verfahren unter Nutzung einer Vorrichtung (10) durchgeführt wird, die eingerichtet ist, mittels eines Prozessgases (40) ein Fließbett von Katalysatorträger-Formkörpern zu erzeugen, in welchem die Katalysatorträger-Formkörper elliptisch oder toroidal umlaufen, vorzugsweise toroidal, umfassend die Schritte des

a) Beschickens der Vorrichtung (10) mit porösen Katalysatorträger-Formkörpern und Erzeugens eines Katalysatorträger-Formkörper-Fließbettes mittels eines Prozessgases (40), wobei die Katalysatorträger-Formkörper in dem Fließbett elliptisch oder toroidal umlaufen, vorzugsweise toroidal;

b) Besprühens der Katalysatorträger-Formkörper mit einer Lösung enthaltend eine katalytisch aktive Spezies oder einen Vorläufer davon, wobei die Katalysatorträger-Formkörper in dem Fließbett elliptisch oder toroidal umlaufen;

c) Trocknens der mit der Lösung besprühten Katalysatorträger-Formkörper.

2. Verfahren nach Anspruch 1, **dadurch gekennzeichnet, dass** das Verfahren ferner einen Schritt des Überführens des Vorläufers in eine katalytisch aktive Spezies umfasst.

3. Verfahren nach einem der Ansprüche 1 oder 2, **dadurch gekennzeichnet, dass** einem in eine Prozesskammer (15) der Vorrichtung (10) eingeführten Prozessgas (40) eine umfängliche Strömungskomponente auferlegt wird, indem durch einen Boden (16) der Prozesskammer (15) zusätzliches Prozessgas (61) mit einer schräg nach oben gerichteten Bewegungskomponente in die Prozesskammer (15) eingeführt wird, vorzugsweise im Bereich einer Seitenwand (18) der Prozesskammer (15).

4. Verfahren nach einem der voranstehenden Ansprüche, **dadurch gekennzeichnet, dass** das Besprühen der in dem Fließbett elliptisch oder toroidal umlaufenden Katalysatorträger-Formkörper mit der Lösung mittels einer Ringspaltdüse (50) durchgeführt wird, die eine Sprühwolke (70) versprüht, die parallel zur Ebene des Bodens (16) der Vorrichtung (10) verläuft.

5. Verfahren nach einem der voranstehenden Ansprüche, **dadurch gekennzeichnet, dass** das Prozessgas (40) erwärmt wird, vorzugsweise auf eine Temperatur von größer/gleich 40 °C.

6. Verfahren nach einem der voranstehenden Ansprüche, **dadurch gekennzeichnet, dass** das Prozessgas (40) vor der Einführung in die Prozesskammer (15) mit dem Lösungsmittel der Lösung angereichert wird, vorzugsweise in einem Bereich von 10 bis 50 % des Sättigungsdampfdrucks.

7. Verfahren nach einem der voranstehenden Ansprüche, **dadurch gekennzeichnet, dass** die Lösung als katalytisch aktive Spezies einen Biokatalysator enthält, vorzugsweise ein Enzym, oder als katalytisch aktive Spezies oder als Vorläufer davon eine Metallverbindung eines Metalls enthält, ausgewählt aus der Gruppe bestehend aus den Übergangsmetallen, insbesondere den Edelmetallen und Co-, Ni- und/oder Cu.

8. Verfahren nach Anspruch 7, **dadurch gekennzeichnet, dass** die Lösung als katalytisch aktive Spezies oder als Vorläufer davon eine Pd-Verbindung, und/oder eine Au-Verbindung und/oder eine Ag-Verbindung und/oder eine Pt-Verbindung enthält.

9. Verfahren nach einem der voranstehenden Ansprüche, **dadurch gekennzeichnet, dass** der Katalysatorträger-Formkörper nach dem Besprühen mit der Lösung einem Fixierungsschritt unterworfen wird.

10. Schalenkatalysator, erhältlich durch ein Verfahren nach einem der Ansprüche 1 bis 9; umfassend einen porösen Katalysatorträger-Formkörper mit einer äußeren Schale, in welcher zumindest eine katalytisch aktive Spezies enthalten ist, **dadurch gekennzeichnet, dass** die Konzentration der katalytisch aktiven Spezies über einen Bereich von 90 % der Schalendicke hinweg, wobei der Bereich zur äußeren und inneren Schalengrenze jeweils um 5 % der Schalendicke beabstandet ist, von der mittleren Konzentration an katalytisch aktiver Spezies dieses Bereichs um maximal +/- 20 % abweicht; und dass über die Dicke der Schale des Katalysators hinweg gesehen die maximale Konzentration an katalytisch aktiver Spezies im Bereich der äußeren Schalengrenze liegt und die Konzentration in Richtung der inneren Schalengrenze abfällt.

11. Katalysator nach einem Anspruch 10, **dadurch gekennzeichnet, dass** die katalytisch aktive Spezies ein Biokatalysator ist, vorzugsweise ein Enzym, oder dass die katalytisch aktive Spezies ein Metall in metallischer Form ist, ausgewählt aus der Gruppe der Übergangsmetalle, vorzugsweise aus der Gruppe der Edelmetalle und Co, Ni und/oder Cu.

12. Katalysator nach einem der Ansprüche 10 bis 11, **dadurch gekennzeichnet, dass** der Katalysator als katalytisch aktive Spezies ein, zwei oder mehr voneinander verschiedene Metalle in der Schale enthält, insbesondere die Metalle einer der folgenden Kombinationen: Pd und Ag; Pd und Au; Pd und Pt.

13. Katalysator nach einem der Ansprüche 10 bis 12, **dadurch gekennzeichnet, dass** der Katalysator als katalytisch aktive Spezies Pd und Au enthält und der Anteil des Katalysators an Pd 0,6 bis 2,0 Mass.-% beträgt, bezogen auf die Masse des mit Edelmetall beladenen Katalysatorträgers und wobei das Au/Pd-Atomverhältnis des Katalysators zwischen 0 und 1,2 liegt.

14. Katalysator nach einem der Ansprüche 10 bis 13, **dadurch gekennzeichnet, dass** der Katalysator ein Alkalimetallacetat umfasst, vorzugsweise Kaliumacetat.

15. Katalysator nach einem der Ansprüche 10 bis 14, **dadurch gekennzeichnet, dass** der Katalysatorträger eine Oberfläche von kleiner/gleich 160 m$^2$/g aufweist.

16. Katalysator nach einem der Ansprüche 10 bis 15, **dadurch gekennzeichnet, dass** der Katalysatorträger eine Azidität von zwischen 1 und 150 $\mu$val/g besitzt.

17. Katalysator nach einem der Ansprüche 10 bis 16, **dadurch gekennzeichnet, dass** der Katalysatorträger mit zumindest einem Oxid eines Metalls dotiert ist, ausgewählt aus der Gruppe bestehend aus Zr, Hf, Ti, Nb, Ta, W, Mg, Re, Y und Fe, vorzugsweise mit $ZrO_2$, $HfO_2$ oder $Fe_2O_3$.

18. Katalysator nach einem der Ansprüche 10 bis 17, **dadurch gekennzeichnet, dass** der Katalysator als katalytisch aktive Spezies Pd und Ag enthält und der Anteil des Katalysators an Pd 0,01 bis 1,0 Mass.-% beträgt, bezogen auf die Masse des mit Edelmetall beladenen Katalysatorträgers, wobei das Ag/Pd-Atomverhältnis des Katalysators zwischen 0 bis 10 liegt und wobei es bevorzugt ist, dass die Dicke der Edelmetallschale kleiner als 60 $\mu$m ist.

19. Katalysator nach einem der Ansprüche 10 bis 18, **dadurch gekennzeichnet, dass** der Katalysator als katalytisch aktive Spezies Pd und Pt enthält und der Anteil des Katalysators an Pd 0,05 bis 5 Mass.-% beträgt, bezogen auf die Masse des mit Edelmetall beladenen Katalysatorträgers und wobei das Pd/Pt-Atomverhältnis des Katalysators zwischen 10 und 1 liegt.

20. Katalysator nach einem der Ansprüche 10 bis 19, **dadurch gekennzeichnet, dass** die Schale des Katalysators eine Dicke von kleiner als 300 $\mu$m aufweist oder dass die Schale des Katalysators eine Dicke von zwischen 200 und 2000 $\mu$m aufweist.

21. Verwendung einer Vorrichtung (10), die eingerichtet ist, mittels eines Prozessgases (40) ein Fließbett von Katalysatorträger-Formkörpern zu erzeugen, in welchem die Katalysatorträger-Formkörpern elliptisch oder toroidal umlaufen, vorzugsweise toroidal, zur Durchführung eines Verfahrens nach einem der voranstehenden Ansprüche 1 bis 9 oder bei der Herstellung eines Schalenkatalysators nach einem der Ansprüche 10 bis 20.


**Claims**

1. Method for producing a shell catalyst which comprises a porous shaped catalyst support body having an outer shell within which at least one catalytically active species is present, the method being performed using a device (10) which is designed to generate, by means of a process gas (40), a fluidized bed of shaped catalyst support bodies in which the shaped catalyst support bodies circulate elliptically or toroidally, preferably toroidally, comprising the steps of

    a) charging the device (10) with porous shaped catalyst support bodies and generating a shaped catalyst support body fluidized bed by means of a process gas (40), the shaped catalyst support bodies circulating elliptically or toroidally in the fluidized bed, preferably toroidally;
    b) spraying the shaped catalyst support bodies with a solution comprising a catalytically active species or a precursor thereof, the shaped catalyst support bodies circulating elliptically or toroidally in the fluidized bed;
    c) drying the shaped catalyst support bodies sprayed with the solution.

2. Method according to Claim 1, **characterized in that** the method further comprises a step of converting the precursor to a catalytically active species.

3. Method according to one of Claims 1 or 2, **characterized in that** a process gas (40) fed into a process chamber (15) of the device (10) is subjected to a circumferential flow component by feeding additional process gas (61), with a movement component directed diagonally upwards, through a bottom (16) of the process chamber (15) into the process chamber (15), preferably in the area of a side wall (18) of the process chamber (15) .

4. Method according to one of the preceding claims, **characterized in that** the spraying of the shaped catalyst support bodies circulating elliptically or toroidally within the fluidized bed with the solution is carried out by means of an annular gap nozzle (50) which atomizes a spray cloud (70) which runs parallel to the plane of the bottom (16) of the device (10).

5. Method according to one of the preceding claims, **characterized in that** the process gas (40) is heated, preferably to a temperature of more than/equal to 40°C.

6. Method according to one of the preceding claims, **characterized in that** the process gas (40), before being fed into the process chamber (15), is enriched with the solvent of the solution, preferably within a range of from 10 to 50% of the saturation vapour pressure.

7. Method according to one of the preceding claims, **characterized in that** the solution comprises a biocatalyst as catalytically active species, preferably an enzyme, or comprises a metal compound of a metal selected from the group consisting of the transition metals, especially the noble metals and Co, Ni and/or Cu, as catalytically active species or as precursor thereof.

8. Method according to Claim 7, **characterized in that** the solution comprises a Pd compound and/or an Au compound and/or an Ag compound and/or a Pt compound as catalytically active species or as precursor thereof.

9. Method according to any of the preceding claims, **characterized in that** the shaped catalyst support body, after being sprayed with the solution, is subjected to a fixing step.

10. Shell catalyst obtainable by a method according to any of Claims 1 to 9; comprising a porous shaped catalyst support body with an outer shell in which at least one catalytically active species is present, **characterized in that** the concentration of the catalytically active species varies over an area of 90% of the shell thickness, where the area is at a distance of 5% of the shell thickness from each of the outer and inner shell boundary, differs by a maximum of +/- 20% from the average concentration of catalytically active species of this area; and **in that**, viewed across the thickness of the shell of the catalyst, the maximum concentration of catalytically active species is in the area of the outer shell boundary and the concentration decreases towards the inner shell boundary.

11. Catalyst according to Claim 10, **characterized in that** the catalytically active species is a biocatalyst, preferably an enzyme, or **in that** the catalytically active species is a metal in metallic form, selected from the group of the transition metals, preferably from the group of the noble metals and Co, Ni and/or Cu.

12. Catalyst according to one of Claims 10 to 11, **characterized in that** the catalyst, as the catalytically active species, comprises one, two or more different metals in the shell, especially the metals of one of the following combinations: Pd and Ag; Pd and Au; Pd and Pt.

13. Catalyst according to one of Claims 10 to 12, **characterized in that** the catalyst comprises, as the catalytically active species, Pd and Au, and the proportion of Pd in the catalyst is 0.6 to 2.0% by mass, based on the mass of the catalyst support laden with noble metal, and wherein the Au/Pd atomic ratio of the catalyst is between 0 and 1.2.

14. Catalyst according to one of Claims 10 to 13, **characterized in that** the catalyst comprises an alkali metal acetate, preferably potassium acetate.

15. Catalyst according to one of Claims 10 to 14, **characterized in that** the catalyst support has a surface area of less than/equal to 160 $m^2$/g.

16. Catalyst according to one of Claims 10 to 15, **characterized in that** the catalyst support has an acidity of between

1 and 150 μeq/g.

**17.** Catalyst according to one of Claims 10 to 16, **characterized in that** the catalyst support is doped with at least one oxide of a metal selected from the group consisting of Zr, Hf, Ti, Nb, Ta, W, Mg, Re, Y and Fe, preferably with $ZrO_2$, $HfO_2$ or $Fe_2O_3$.

**18.** Catalyst according to one of Claims 10 to 17, **characterized in that** the catalyst comprises Pd and Ag as the catalytically active species and the proportion of Pd in the catalyst is 0.01 to 1.0% by mass, based on the mass of the catalyst support laden with noble metal, wherein the Ag/Pd atomic ratio of the catalyst is between 0 and 10 and wherein it is preferred that the thickness of the noble metal shell is less than 60 μm.

**19.** Catalyst according to one of Claims 10 to 18, **characterized in that** the catalyst comprises Pd and Pt as catalytically active species and the proportion of Pd in the catalyst is 0.05 to 5% by mass, based on the mass of the catalyst support laden with noble metal, and wherein the Pd/Pt atomic ratio of the catalyst is between 10 and 1.

**20.** Catalyst according to one of Claims 10 to 19, **characterized in that** the shell of the catalyst has a thickness of less than 300 μm or **in that** the shell of the catalyst has a thickness of between 200 and 2000 μm.

**21.** Use of a device (10) which is designed to generate a fluidized bed of shaped catalyst support bodies by means of a process gas (40), in which the shaped catalyst support bodies circulate elliptically or toroidally, preferably toroidally, to perform a method according to any of the preceding Claims 1 to 9 or in the production of a shell catalyst according to any of Claims 10 to 20.

## Revendications

**1.** Procédé de fabrication d'un catalyseur à enveloppe, qui comprend un corps moulé support de catalyseur poreux muni d'une enveloppe extérieure, dans laquelle au moins une espèce catalytiquement active est contenue, le procédé étant réalisé en utilisant un dispositif (10) qui est conçu pour former un lit fluidisé de corps moulés supports de catalyseur au moyen d'un gaz de procédé (40), dans lequel les corps moulés supports de catalyseur sont mis en circulation sous une forme elliptique ou toroïdale, de préférence toroïdale, comprenant les étapes suivantes :

a) le chargement du dispositif (10) avec des corps moulés supports de catalyseur poreux et la formation d'un lit fluidisé de corps moulés supports de catalyseur au moyen d'un gaz de procédé (40), les corps moulés supports de catalyseur étant mis en circulation sous forme elliptique ou toroïdale, de préférence toroïdale, dans le lit fluidisé ;
b) la pulvérisation des corps moulés supports de catalyseur avec une solution contenant une espèce catalytiquement active ou un précurseur de celle-ci, les corps moulés supports de catalyseur étant mis en circulation sous forme elliptique ou toroïdale dans le lit fluidisé ;
c) le séchage des corps moulés supports de catalyseur pulvérisés avec la solution.

**2.** Procédé selon la revendication 1, **caractérisé en ce que** le procédé comprend en outre une étape de transformation du précurseur en une espèce catalytiquement active.

**3.** Procédé selon l'une quelconque des revendications 1 ou 2, **caractérisé en ce qu'**un composant d'écoulement circonférentiel est appliqué à un gaz de procédé (40) introduit dans une chambre de procédé (15) du dispositif (10), par introduction d'un gaz de procédé supplémentaire (61) dans la chambre de procédé (15) au travers d'un fond (16) de la chambre de procédé (15) avec un composant de mouvement orienté en biais vers le haut, de préférence dans la zone d'une paroi latérale (18) de la chambre de procédé (15).

**4.** Procédé selon l'une quelconque des revendications précédentes, **caractérisé en ce que** la pulvérisation des corps moulés supports de catalyseur en circulation sous forme elliptique ou toroïdal dans le lit fluidisé avec la solution est réalisée au moyen d'une buse à fente annulaire (50), qui pulvérise un nuage de pulvérisation (70), qui est parallèle au plan du fond (16) du dispositif (10) .

**5.** Procédé selon l'une quelconque des revendications précédentes, **caractérisé en ce que** le gaz de procédé (40) est chauffé, de préférence à une température supérieure ou égale à 40 °C.

**6.** Procédé selon l'une quelconque des revendications précédentes, **caractérisé en ce que** le gaz de procédé (40) est enrichi avec le solvant de la solution avant l'introduction dans la chambre de procédé (15), de préférence dans une plage allant de 10 à 50 % de la pression de vapeur de saturation.

**7.** Procédé selon l'une quelconque des revendications précédentes, **caractérisé en ce que** la solution contient en tant qu'espèce catalytiquement active un biocatalyseur, de préférence une enzyme, ou contient en tant qu'espèce catalytiquement active ou en tant que précurseur de celle-ci un composé métallique d'un métal, choisi dans le groupe constitué par les métaux de transition, notamment les métaux nobles et Co, Ni et/ou Cu.

**8.** Procédé selon la revendication 7, **caractérisé en ce que** la solution contient en tant qu'espèce catalytiquement active ou en tant que précurseur de celle-ci un composé de Pd et/ou un composé d'Au et/ou un composé d'Ag et/ou un composé de Pt.

**9.** Procédé selon l'une quelconque des revendications précédentes, **caractérisé en ce que** le corps moulé support de catalyseur est soumis à une étape de fixation après la pulvérisation avec la solution.

**10.** Catalyseur à enveloppe, pouvant être obtenu par un procédé selon l'une quelconque des revendications 1 à 9 ; comprenant un corps moulé support de catalyseur poreux muni d'une enveloppe extérieure, dans laquelle au moins une espèce catalytiquement active est contenue, **caractérisé en ce que** la concentration de l'espèce catalytiquement active diffère sur une zone de 90 % de l'épaisseur de l'enveloppe, la zone étant espacée à chaque fois de 5 % de l'épaisseur de l'enveloppe de la limite extérieure et de la limite intérieure de l'enveloppe, d'au plus $\pm$ 20 % de la concentration moyenne en espèce catalytiquement active de cette zone ; et
**en ce que**, sur l'épaisseur de l'enveloppe du catalyseur, la concentration maximale en espèce catalytiquement active se situe dans la zone de la limite extérieure de l'enveloppe et la concentration diminue dans la direction de la limite intérieure de l'enveloppe.

**11.** Catalyseur selon la revendication 10, **caractérisé en ce que** l'espèce catalytiquement active est un biocatalyseur, de préférence une enzyme, ou **en ce que** l'espèce catalytiquement active est un métal sous forme métallique, choisi dans le groupe constitué par les métaux de transition, de préférence dans le groupe constitué par les métaux nobles et Co, Ni et/ou Cu.

**12.** Catalyseur selon l'une quelconque des revendications 10 à 11, **caractérisé en ce que** le catalyseur contient en tant qu'espèce catalytiquement active un, deux ou davantage de métaux différents les uns des autres dans l'enveloppe, notamment les métaux d'une des combinaisons suivantes : Pd et Ag ; Pd et Au ; Pd et Pt.

**13.** Catalyseur selon l'une quelconque des revendications 10 à 12, **caractérisé en ce que** le catalyseur contient en tant qu'espèce catalytiquement active Pd et Au, et la proportion du catalyseur en Pd est de 0,6 à 2,0 % en masse, par rapport à la masse du support de catalyseur chargé avec un métal noble, et le rapport atomique Au/Pd du catalyseur est compris entre 0 et 1,2.

**14.** Catalyseur selon l'une quelconque des revendications 10 à 13, **caractérisé en ce que** le catalyseur comprend un acétate de métal alcalin, de préférence l'acétate de potassium.

**15.** Catalyseur selon l'une quelconque des revendications 10 à 14, **caractérisé en ce que** le support de catalyseur présente une surface inférieure ou égale à 160 m$^2$/g.

**16.** Catalyseur selon l'une quelconque des revendications 10 à 15, **caractérisé en ce que** le support de catalyseur présente une acidité comprise entre 1 et 150 pval/g.

**17.** Catalyseur selon l'une quelconque des revendications 10 à 16, **caractérisé en ce que** le support de catalyseur est dopé avec au moins un oxyde d'un métal, choisi dans le groupe constitué par Zr, Hf, Ti, Nb, Ta, W, Mg, Re, Y et Fe, de préférence avec $ZrO_2$, $HfO_2$ ou $Fe_2O_3$.

**18.** Catalyseur selon l'une quelconque des revendications 10 à 17, **caractérisé en ce que** le catalyseur contient en tant qu'espèce catalytiquement active Pd et Ag, et la proportion du catalyseur en Pd est de 0,01 à 1,0 % en masse, par rapport à la masse du support de catalyseur chargé avec un métal noble, le rapport atomique Ag/Pd du catalyseur étant compris entre 0 et 10, et l'épaisseur de l'enveloppe de métal noble étant de préférence inférieure à 60 $\mu$m.

**19.** Catalyseur selon l'une quelconque des revendications 10 à 18, **caractérisé en ce que** le catalyseur contient en tant qu'espèce catalytiquement active Pd et Pt, et la proportion du catalyseur en Pd est de 0,05 à 5 % en masse, par rapport à la masse du support de catalyseur chargé avec un métal noble, le rapport atomique Pd/Pt du catalyseur étant compris entre 10 et 1.

**20.** Catalyseur selon l'une quelconque des revendications 10 à 19, **caractérisé en ce que** l'enveloppe du catalyseur présente une épaisseur inférieure à 300 pm, ou **en ce que** l'enveloppe du catalyseur présente une épaisseur comprise entre 200 et 2 000 $\mu$m.

**21.** Utilisation d'un dispositif (10) qui est conçu pour former un lit fluidisé de corps moulés supports de catalyseur au moyen d'un gaz de procédé (40), dans lequel les corps moulés supports de catalyseur sont mis en circulation sous forme elliptique ou toroïdale, de préférence toroïdale, pour la réalisation d'un procédé selon l'une quelconque des revendications 1 à 9 précédentes, ou lors de la fabrication d'un catalyseur à enveloppe selon l'une quelconque des revendications 10 à 20.

**Fig. 1A**

EP 2 155 382 B1

Fig. 18

Fig. 2A

**Fig. 2B**

**Fig. 3A**

310

Fig. 3B

310

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- EP 565952 A1
- EP 634214 A1
- EP 634209 A1
- EP 634208 A1
- WO 9962632 A1
- WO 2005061107 A1
- WO 2006027009 A1
- DE 10248116 B3
- EP 0370167 A1
- EP 0436787 B1
- DE 19904147 A1
- DE 202005003791 U1
- WO 02100527 A1

### In der Beschreibung aufgeführte Nicht-Patentliteratur

- Lehrbuch der anorganischen Chemie. Hollemann Wiberg, de Gruyter, 2007
- Römpp Lexikon Chemie. Georg Thieme Verlag
- **RÖMPP.** Lexikon Chemie. Georg Thieme Verlag
- *J. Am. Chem. Soc.,* 1938, vol. 60, 309
- **E.P. BARRET ; L.G. JOINER ; P.P. HAIENDA.** *J. Am. Chem. Soc.,* 1951, vol. 73, 373